# EUROPEAN PATENT APPLICATION

(11) **EP 4 582 421 A2**
(43) Date of publication of application: **09.07.2025**
(21) Application number: 25177219.0
(22) Date of filing: 07.04.2022
(51) Int. Cl.: C07D 405/14

(54) **GLP-1 RECEPTOR AGONIST, PHARMACEUTICAL COMPOSITION COMPRISING SAME, AND METHOD FOR PREPARING SAME**

(30) Priority: 08.04.2021 KR 20210046024
(62) Divisional of application: 22785003.9
(71) Applicant: LG Chem, Ltd., Seoul 07336 (KR)
(72) Inventor: Kim, Young Kwan, 34122 Daejeon (KR); Park, Jun, 34122 Daejeon (KR); Jo, Min Mi, 34122 Daejeon (KR)
(74) Representative: Hoffmann Eitle

(57) **Abstract**

The present invention relates to: a novel compound having excellent GLP-1 agonist activity and an excellent DMPK profile, and thus useful as an agent for treating or preventing obesity or various metabolic diseases such as diabetes and hyperlipidemia; an isomer thereof or a pharmaceutically acceptable salt thereof; a pharmaceutical composition comprising the compound; and a method for preparing the compound.

## Description

### FIELD OF INVENTION

The present disclosure relates to a new compound exhibiting Glucagon-like peptide-1 (GLP-1) receptor agonist activity, an isomer thereof, or a pharmaceutically acceptable salt thereof, a pharmaceutical composition containing the compound, and a method for preparing the compound.

### BACKGROUND

Glucagon-like peptide-1 (GLP-1) is a polypeptide hormone secreted by intestinal L-cells after meals and may stimulate insulin secretion from pancreatic islet β cells, thereby stabilizing postprandial blood glucose levels. This GLP-1 binds to the GLP-1 receptor (GLP-1R). The GLP-1 receptor is a protein belonging to the Class B receptor subclass of the G protein-coupled receptor (GPCR) that regulates important physiological and pathophysiological processes, and protein tertiary structure is not characterized, since the receptor N-terminus has a unique binding mode to bind ligands to determine affinity, it is recognized as a drug target that is very difficult to develop small molecule synthetic ligands.

Exogenous administration of GLP-1 normalizes blood glucose levels in patients with type 2 diabetes. Since the effect of GLP-1 on lowering blood glucose levels depends on the glucose concentration, it greatly reduces the risk of hypoglycemia while controlling blood glucose levels. In addition, drugs based on GLP-1, such as Byetta^{®} and Bydureon BCise^{®} (exenatide), Ozempic^{®} (semaglutide), Victoza^{®} (liraglutide), Adlyxin^{®} (lixisenatide); Tanzeum^{®} (albiglutide), and Trulicity^{®} (dulaglutide), are GLP-1 receptor agonists and have been successfully marketed in recent years, for example, it has been found to provide effective glycemic control for the treatment of patients with type 2 diabetes, in addition to providing a weight loss effect, preservation of beta-cell function and alleviation of hypertension, hypoglycemia and/or hyperlipidemia.

However, there is a need for small molecule agonists of GLP-1 receptors with oral bioavailability since the mentioned GLP-1 and GLP-1 receptor agonists may lack sufficient oral bioavailability to be considered as peptide-based oral drugs.

### SUMMARY

As proposed to solve the above problems, the present disclosure provides a new compound having activity as a GLP-1 agonist.

In addition, the present disclosure provides a pharmaceutical composition for preventing or treating metabolic diseases or neurodegenerative diseases comprising the new compound as an active ingredient.

### DEFINITIONS

The definition of each group used in the present specification will be described in detail. Unless otherwise specified, each group has the following definitions.

In the present specification, examples of "halo" may be fluoro, chloro, bromo or iodo.

In the present specification, "alkyl" refers to a straight or branched aliphatic saturated hydrocarbon group, and specifically, it may be 1 to 6 carbon atoms, namely, C₁₋₆ alkyl, C₁₋₄ alkyl, or C₁₋₃ alkyl. Examples of such alkyl may be methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethyl butyl, 2,2-dimethylbutyl, 3,3-dimethylbutyl or 2-ethylbutyl.

In the present specification, "alkoxy" refers to an oxygen group to which a single bond straight or branched saturated hydrocarbon is bonded, and may specifically be C₁₋₆ alkoxy, C₁₋₄ alkoxy, or C₁₋₃ alkoxy. Examples of such alkoxy may be methoxy, ethoxy, propoxy, n-butoxy, tert-butoxy or 1-methylpropoxy.

In the present specification, "cycloalkyl" refers to a saturated hydrocarbon group of a cyclic single bond, and specifically, may be C₃₋₈ cycloalkyl or C₃₋₆ cycloalkyl depending on the number of carbon atoms. Examples of such cycloalkyl include cyclopropyl, cyclobutyl, cyclopentyl or cyclohexyl.

In the present specification, "cycloalkyl" may be a fused or bridged of two or more cycloalkyls; specifically, it may be a bridged bi-cycloalkyl, a bridged tri-cycloalkyl; it may encompass prismaines and asteranes such as prismane, cubane, basketane, and the like; and examples of such bridged cycloalkyls may include bicyclo[1,1,0]butane, bicyclo[1,1,1]pentane, bicyclo[2,1,1]hexane, bicyclo[2,2,1]heptane, bicyclo[2,2,2]octane, and the like.

In the present specification, "heterocycloalkyl" refers to a saturated hydrocarbon group of a cyclic single bond containing one or more heteroatoms such as N, O, or S in addition to carbon atoms as ring constituent atoms, and may be monocyclic or fused ring polycyclic. Specifically, it may be 4- to 10-membered heterocycloalkyl, 4- to 7-membered heterocycloalkyl, or 4- to 6-membered heterocycloalkyl containing one or more, preferably 1 to 3 heteroatoms selected from the group consisting of N, O and S. Examples of such heterocycloalkyl may include oxytanyl, aziridine, pyrrolidine, pyrrolidinyl, piperidinyl, piperazinyl, morpholinyl, tetrahydrofuranyl or tetrahydropyranyl.

In the present specification, "aryl" refers to an aromatic substituent having at least one ring having a shared pi electron system, and may be monocyclic or fused ring polycyclic (namely, rings that share adjacent pairs of carbon atoms). Specifically, the aryl may be C₄₋₁₀ aryl or C₆₋₁₀ aryl, depending on the number of carbon atoms included in the ring, for example, phenyl or naphthyl.

In the present specification, "heteroaryl" refers to an aromatic ring compound containing one or more heteroatoms such as N, O, or S in addition to carbon atoms as ring constituent atoms, and may be monocyclic or fused ring polycyclic. Specifically, it may be 4- to 10-membered heteroaryl, 4- to 7-membered heteroaryl, or 4- to 6-membered heteroaryl containing one or more, preferably 1 to 3 heteroatoms selected from the group consisting of N, O and S. Examples of the heteroaryl include furanyl, pyranyl, imidazolyl, oxazolyl, isoxazolyl, pyridyl, pyrazinyl, pyrimidyl, pyridazinyl, oxadiazolyl, thiadiazolyl, tetrazolyl, triazinyl, triazyl, triazolyl, and the like, but are not limited thereto.

In the present specification, the "substituent" may be one or more selected from the group consisting of halo, a nitrile group, and a C₁₋₃ alkyl group.

Hereinafter, the present disclosure will be described in more detail.

### DETAILED DESCRIPTION

In order to achieve the above aspect, the present disclosure provides a compound of the following Chemical Formula I, an isomer thereof, or a pharmaceutically acceptable salt thereof. 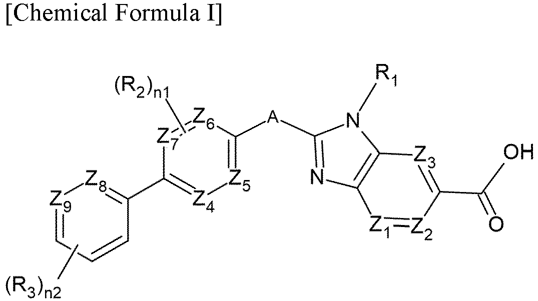
A is -(CH₂)ₘ-, -O- or -N(Rₐ)-, wherein m is an integer from 1 to 3 and Rₐ is hydrogen or alkyl;
Z₁, Z₂, Z₃, Z₄, Z₅, Z₆ or Z₇ each independently represents CH, CF, CCl, CBr, CI or N;
Z₈ or Z₉ is each independently C or N substituted with an -O-CH₂-R group, or when Z₈ and Z₉ are both C, each substituent may be condensed with each other to form a dioxole structure;
R is cycloalkyl or 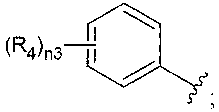
R₁ is (cycloalkyl)alkyl, (heterocycloalkyl)alkyl, (aryl)alkyl or (heteroaryl)alkyl;
R₂, R₃ or R₄ is each independently a hydrogen, deuterium, halo, alkyl, alkoxy, alkylamine or nitrile group;
n₁ to n₃ are each independently an integer of 1 to 4, wherein, when n₁ to n₃ are an integer of 2 or more, each of R₂, R₃ or R₄ may be the same as or different from each other; and
at this time, the alkyl, alkoxy, alkylamine, cycloalkyl, heterocycloalkyl, aryl, or heteroaryl is unsubstituted or substituted.

Specifically, the compound of the following Chemical Formula I may be a compound represented by Chemical Formula 1, Chemical Formula 1' or Chemical Formula 1", an isomer thereof, or a pharmaceutically acceptable salt thereof: 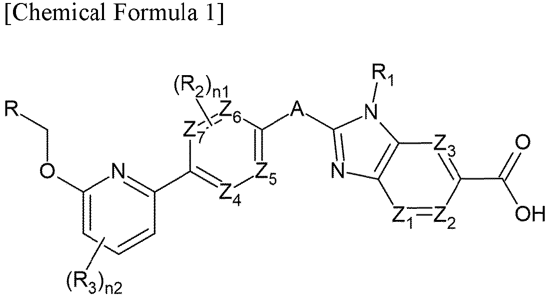 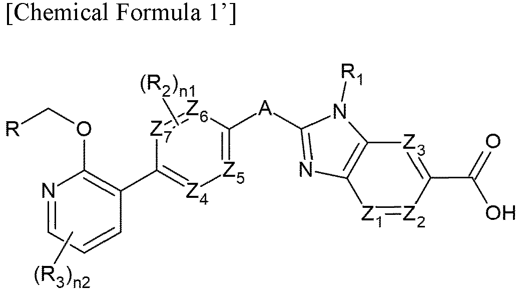 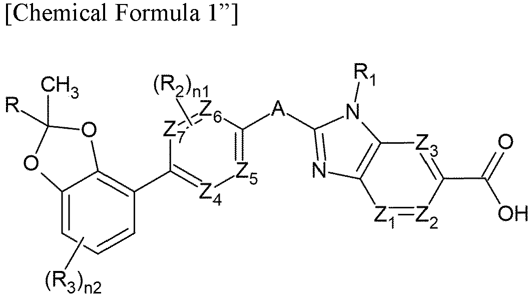 A, Z₁, Z₂, Z₃, Z₄, Z₅, Z₆, Z₇, R, R₁, R₂, R₃, R₄ and n₁ to n₃ are as defined in claim 1.

In a preferred aspect, A may be -CH₂-, -O- or -N(Rₐ)-.

In a preferred aspect, Rₐ may be hydrogen or C₁₋₃ alkyl.

In a preferred aspect, R may be C₃₋₈ cycloalkyl or 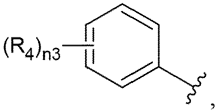 **and** C₃₋₈ cycloalkyl may be bridged or unbridged cycloalkyl, may include mono-, bi-, poly- and bridged ring systems, but is not limited thereto.

In a preferred aspect, R₁ may be (C₃₋₈ cycloalkyl)C₁₋₃ alkyl, (4- to 10-membered heterocycloalkyl)C₁₋₃ alkyl, (C₆₋₁₀ aryl)alkyl or (4- to 10-membered heteroaryl)C₁₋₃ alkyl, wherein the heterocycloalkyl or heteroaryl contains 1 to 3 heteroatoms selected from the group consisting of N, O and S.

In a preferred aspect, R₂, R₃ or R₄ are each independently hydrogen, deuterium, F, Cl, Br, I, C₁₋₃ alkyl, C₁₋₃ alkoxy, C₁₋₃ alkylamine or a nitrile group.

In a preferred aspect, n₁ to n₃ are each independently an integer of 1 to 4, wherein, when n₁ to n₃ are an integer of 2 or more, each of R₂, R₃ or R₄ may be the same as or different from each other.

In a preferred aspect, Z₁, Z₂, Z₃, Z₄, Z₅, Z₆ or Z₇ may each independently be CH, CF or CCl.

In a preferred aspect, the alkyl, alkoxy, alkylamine, cycloalkyl, heterocycloalkyl, aryl, or heteroaryl may be unsubstituted or substituted with halo or C₁₋₃ alkyl.

In a more preferred aspect, A may be -CH₂- or -O-.

In a more preferred aspect, R may be bicyclo[1,1,0]butane, bicyclo[1,1,1]pentane, bicyclo[2,1,1]hexane, bicyclo[2,2,1]heptane, bicyclo[2,2,2]octane or

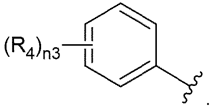

In a more preferred aspect, R₁ may be cyclopropylmethyl, cyclobutylmethyl, cyclopentylmethyl, cyclohexylmethyl, oxytanylmethyl, tetrahydrofuranylmethyl, tetrahydropyranylmethyl, oxazolylmethyl, benzyl, unsubstituted or triazolylmethyl substituted with propyl, or unsubstituted or imidazolylmethyl substituted with ethyl.

In a more preferred aspect, R₂, R₃ or R₄ are each independently hydrogen, deuterium, F, Cl, or a nitrile group.

In a more preferred aspect, n₁ to n₃ are 2, and each of R₂, R₃ or R₄ may be the same as or different from each other.

Representative compounds of Chemical Formula I according to the present disclosure may include, but are not limited to:
1] (*S*)-2-(4-(2-((4-chloro-2-fluorobenzyl)oxy)pyridin-3-yl)benzyl)-1-(oxytan-2-ylmethyl)-1*H*-benzo[*d*]imidazol-6-carboxylic acid;
2] 2-(4-(2-(4-chloro-2-fluorophenyl)-2-methylbenzo[*d*][1,3]dioxol-4-yl)benzyl)-1-(((*S*)-oxytan-2-yl)methyl)-1*H*-benzo[*d*]imidazol-6-carboxylic acid;
3] 2-(4-(2-(4-chloro-2-fluorophenyl)-2-methylbenzo[*d*][1,3]dioxol-4-yl)-3-fluorobenzyl)-1-(((*S*)-oxytan-2-yl)methyl)-1*H*-benzo[*d*]imidazol-6-carboxylic acid;
4] 2-(4-(2-(4-chloro-2-fluorophenyl)-2-methylbenzo[*d*][1,3]dioxol-4-yl)benzyl)-1-(oxazol-5-ylmethyl)-1*H*-benzo[d]imidazol-6-carboxylic acid;
5] 2-(4-(2-(4-chloro-2-fluorophenyl)-2-methylbenzo[*d*][1,3]dioxol-4-yl)-3-fluorobenzyl)-1-(oxazol-5-ylmethyl)-1*H*-benzo[*d*]imidazol-6-carboxylic acid;
6] 2-(4-(2-((4-chloro-2-fluorobenzyl)oxy)pyridin-3-yl)benzyl)-1-(oxazol-5-ylmethyl)-1*H*-benzo[*d*]imidazol-6-carboxylic acid;
7] 2-(4-(2-((4-chloro-2-fluorobenzyl)oxy)pyridin-3-yl)benzyl)-1-((4-propyl-4*H*-1,2,4-triazol-3-yl)methyl)-1*H*-benzo[d]imidazol-6-carboxylic acid;
8] 2-(4-(2-(4-chloro-2-fluorophenyl)-2-methylbenzo[*d*][1,3]dioxol-4-yl)benzyl)-1-((4-propyl-4*H*-1,2,4-triazol-3-yl)methyl)-1*H*-benzo[d]imidazol-6-carboxylic acid;
9] 2-(4-(2-(4-chloro-2-fluorophenyl)-2-methylbenzo[*d*][1,3]dioxol-4-yl)-3-fluorobenzyl)-1-((4-propyl-4*H*-1,2,4-triazol-3-yl)methyl)-1*H*-benzo[*d*]imidazol-6-carboxylic acid;
10] 2-(4-(2-(4-chloro-2-fluorophenyl)-2-methylbenzo[*d*][1,3]dioxol-4-yl)benzyl)-1-((1-ethyl-1*H*-imidazol-5-yl)methyl)-1*H*-benzo[d]imidazol-6-carboxylic acid;
11] 2-(4-(2-(4-chloro-2-fluorophenyl)-2-methylbenzo[*d*][1,3]dioxol-4-yl)-3-fluorobenzyl)-1-((1-ethyl-1*H*-imidazol-5-yl)methyl)-1*H*-benzo[d]imidazol-6-carboxylic acid;
12] 2-(4-(2-(4-chloro-2-fluorophenyl)-2-methylbenzo[*d*][1,3]dioxol-4-yl)benzyl)-1-(((S)-tetrahydrofuran-2-yl)methyl)-1*H*-benzo[*d*]imidazol-6-carboxylic acid; and
13] (*S*)-2-(4-(6-(bicyclo[2.2.2]octan-1-ylmethoxy)pyridin-2-yl)benzyl)-1-(oxytan-2-ylmethyl)-1*H*-benzo[d]imidazol-6-carboxylic acid.

Compounds belonging to the above-described category of Chemical Formula I may exhibit excellent GLP-1 receptor agonist activity, and thus exhibit a hypoglycemic action and a positive effect on pancreatic beta-cells, so that they may be used more effectively to treat various metabolic diseases.

Meanwhile, the compound represented by the Chemical Formula I may have an asymmetric carbon center, and when it has an asymmetric carbon center, it may exist as individual optical isomers, partial optical isomers, or racemates, and all forms of isomers including these also may be included in the category of compounds according to one embodiment of the present disclosure. It goes without saying that any form of an isomer may also fall within the scope of the compound of one embodiment. As used hereinafter, the term "isomer" may generically refer to different compounds having the same molecular formula, and "optical isomer" may collectively refer to any stereoisomers that may exist for a compound of one embodiment, including the same geometric isomer.

It is understood that in the compound represented by Chemical Formula I according to one embodiment, each substituent may be attached to a chiral center of a carbon atom. And, any asymmetric carbon atom on the compound of one embodiment may exist in any form of *(R)-,* (S)- or (R, *S*)- configuration, suitably in each isolated form (R)- or (S)- configuration. In addition, the compound of one embodiment may exist in any form of any possible isomer or mixture thereof, for example, in any form of pure geometric isomer, diastereomer, optical isomer, racemate or mixtures thereof. In addition, when the compound of one embodiment has a double bond, each substituent bonded to the double bond may have an E or Z configuration. Also, when the compound of one embodiment contains a disubstituted cycloalkyl, each substituent of the cycloalkyl may have a cis or trans configuration.

Meanwhile, the term "pharmaceutically acceptable salt" as used hereinafter has the same biological effectiveness and properties of the compound of Chemical Formula I according to one embodiment, and may collectively refer to any salt that is desirable in terms of pharmaceutical, biological, or other properties. Non-limiting examples of such salts include salts in which an inorganic or organic base was added to a compound of Chemical Formula I, or acid addition salts. Examples of organic acids capable of forming such acid addition salts include acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid or salicylic acid, and the like, and examples of the inorganic acid include hydrochloric acid, hydrobromic acid, sulfuric acid, nitric acid or phosphoric acid.

The pharmaceutically acceptable salt of the compound of the above-described embodiment may be synthesized from the free base form of the compound or any basic or acidic moiety derived therefrom by chemical methods in the related art. Additionally, a second pharmaceutically acceptable salt may be synthesized from a first pharmaceutically acceptable salt. As a specific example, an acid addition salt of a compound of one embodiment may be obtained by reacting a compound in its free base form with a stoichiometric amount of an appropriate acid. In this case, the reaction may be carried out in water, an organic solvent, or a mixture thereof, specifically, in a non-aqueous medium such as ether, ethyl acetate, ethanol, isopropanol or acetonitrile. In addition, depending on the form of the pharmaceutically acceptable salt, each form of the salt may be obtained by a conventional reaction obvious to those skilled in the art.

Meanwhile, according to another embodiment of the present disclosure, there is provided a pharmaceutical composition for the treatment or prevention of metabolic diseases, comprising the compound represented by the above-described Chemical Formula I, an isomer thereof, or a pharmaceutically acceptable salt thereof as an active ingredient. As described above, the compounds represented by Chemical Formula I according to the present disclosure exhibit GLP-1 receptor (GLP-1R) agonistic activity, and a pharmaceutical composition containing such a compound may exhibit an effect of improving lipid metabolism which is a chronic cardiovascular risk factor while having an effective hypoglycemic action and a positive effect on pancreatic beta-cell activity, so that it is effective in the treatment and/or prophylaxis of diseases which are associated with the activity of the GLP-1 receptor, for example metabolic diseases or neurodegenerative diseases. The metabolic disease may be a disease selected from the group consisting of diabetes (preferably type 2 diabetes), hypertension, hypoglycemia, hyperlipidemia (dyslipidemia), atherosclerosis, coronary artery disease, cardiovascular disorder, blood coagulation abnormality, obesity, diabetic complications, diabetic retinopathy, liver disease, hepatobiliary disease, fatty liver, alcoholic steatohepatitis, chronic kidney disease, insulin resistance, and impaired glucose tolerance. The neurodegenerative disease may be a disease selected from the group consisting of Parkinson's disease and Alzheimer's disease.

The pharmaceutical composition containing the compound represented by Chemical Formula I, an isomer thereof, or a pharmaceutically used salt thereof as an active ingredient may be used in the form of a pharmaceutical preparation in the related art. That is, the pharmaceutical composition may be administered in various oral or parenteral formulation during actual clinical administration, and may be appropriately administered in an oral formulation. In addition, according to each formulation, fillers, extenders, binders, wetting agents, disintegrants, or surfactants in the related art such as pharmaceutically acceptable diluents or excipients may be further included in the formulation.

Solid preparations for oral administration may include tablets, pills, powders, granules, or capsules, and these solid preparations may be provided by mixing starch, calcium carbonate, sucrose or lactose, gelatin, and the like with the active ingredient. Also, in addition to the excipient, a lubricant such as magnesium stearate or talc may be used. In addition, liquid preparations for oral administration may include suspensions, internal solutions, emulsions or syrups, and these liquid preparations may include water which is a simple diluent, or liquid paraffin, but also various excipients, for example, wetting agents, sweeteners, fragrances or preservatives, and the like. In addition, preparations for parenteral administration include sterile aqueous solutions, non-aqueous solutions, suspensions, emulsions, lyophilized preparations or suppositories. The parenteral preparation may include a non-aqueous solvent, and the suspension solution may include propylene glycol, polyethylene glycol, vegetable oil such as olive oil, or injectable ester such as ethyl oleate. As the suppository base, witepsol, Macrogol, tween 61, cacao butter, laurin butter, or glycerogeratin, and the like may be used.

In addition, the compound represented by the above Chemical Formula I of the pharmaceutical disclosure containing the present or pharmaceutically acceptable salt thereof as an active ingredient, and an isomer composition thereof may represent an effective amount in an administration range of about 0.1 to about 1,000 mg. The dosing amount or dosage may be administered in a variety of dosages and methods, such as one or several times a day, depending on the patient's weight, age, sex, health status, diet, administration time, administration method, excretion rate, and severity of disease.

The present disclosure also provides a method for preparing a compound represented by Chemical Formula 1, Chemical Formula 1' or Chemical Formula 1".

Hereinafter, a method for preparing a compound represented by Chemical Formula 1, Chemical Formula l' or Chemical Formula 1" will be described based on an exemplary reaction scheme in order to help the understanding of the present disclosure. However, those skilled in the art will be able to prepare compounds of Chemical Formula 1, Chemical Formula 1' or Chemical Formula 1", by a variety of methods, based on the structure of Chemical Formula 1, Chemical Formula 1' or Chemical Formula 1", all of which are to be interpreted as falling within the scope of the present disclosure. That is, it will be understood that the compounds of Chemical Formula 1, Chemical Formula 1' or Chemical Formula 1" may be prepared by any combination of the various synthetic methods described herein or disclosed in the related art, and are not intended to be within the scope of the present disclosure, and that the methods for preparing the compound of Chemical Formula 1, Chemical Formula 1' or Chemical Formula 1" are not limited only to those described below.

In a specific embodiment, when A is carbon in the compound of Chemical Formula 1 according to the present disclosure, the compound of Chemical Formula 1 may be prepared by a preparation method, comprising:
1) reacting a compound of the following Chemical Formula 2 with a compound of the following Chemical Formula 3 under a palladium catalyst to obtain a compound of the following Chemical Formula 4;
2) reacting the compound of the following Chemical Formula 4 obtained in step 1) with a compound of the following Chemical Formula 5 under a palladium catalyst and then hydrolyzing to obtain a compound of the following Chemical Formula 6; and
3) a coupling reaction of the compound of the following Chemical Formula 6 obtained in step 2) with a compound of the following Chemical Formula 7, followed by condensation and hydrolysis to obtain a compound of the following Chemical Formula 1.

The base used in steps 1) and 2) above may be used alone or in combination with a material selected from the group consisting of C₁₋₄ trialkylamine, diisopropylethyleneamine (DIPEA, Hunig's base), pyridine, K₂CO₃, KOH, NaOH, Na₂CO₃, NaOAc, Ca(OH)₂, NaHCO₃, Cs2CO₃ and LiOH, and the ligands used in steps 1) and 2) may be triarylphosphines, trialkylphosphines, biaryl(dialkyl)phosphines, diphosphines, N-heterocyclic carbenes, cyclopentadienides, acetylacetonates, diamines, bipyridines, pyridines, DIOP, DiPAMP, BINAP, chiraphos, and the like, but is not limited thereto.

The hydrolysis reaction of step 2) may be carried out using NaOH, KOH, LiOH, and the like, and may be carried out at a temperature of 0 °C to 80 °C, 10 to 70 °C, 20 to 60 °C, or room temperature or 50 °C, but is not limited thereto. In addition, the reaction may be carried out through stirring for an appropriate time during the reaction, which may be appropriately controlled.

This may be represented by Scheme 1 below.

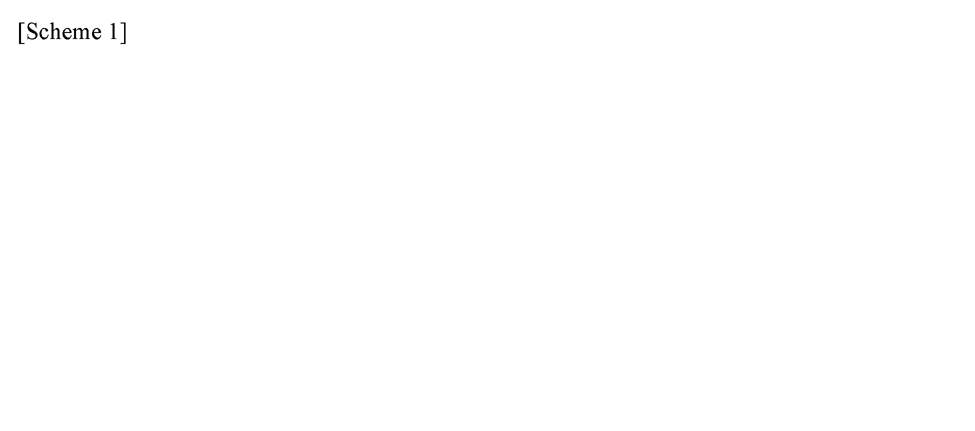

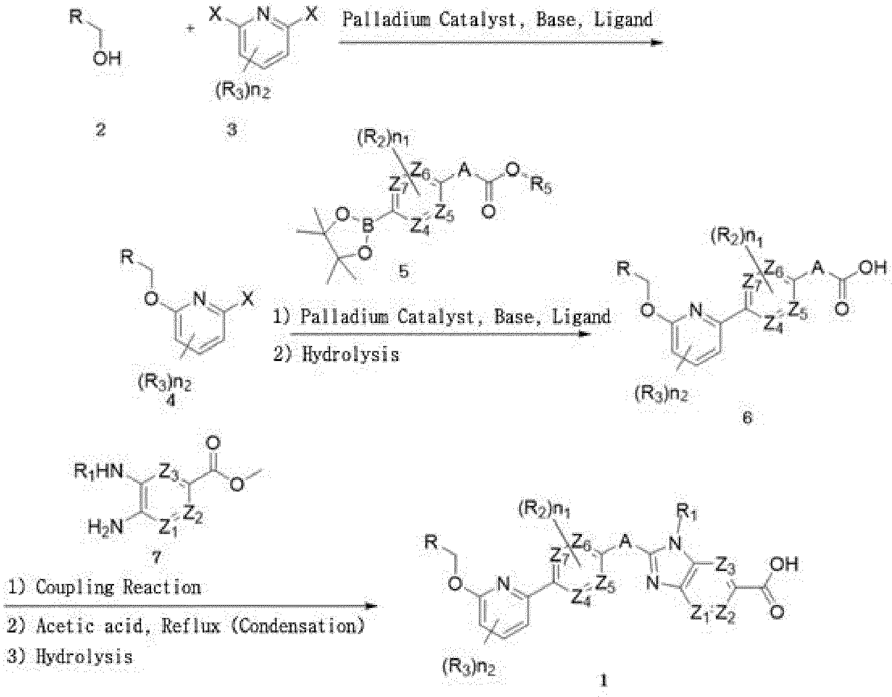

In a specific embodiment, when A is carbon in the compound of Chemical Formula 1' according to the present disclosure, the compound of Chemical Formula 1' may be prepared by a preparation method, comprising:
1') reacting a compound of the following Chemical Formula 3' with a compound of the following Chemical Formula 5 under a palladium catalyst to obtain a compound of the following Chemical Formula 8;
2') reacting the compound of the following Chemical Formula 8 obtained in step 1') with a compound of the following Chemical Formula 2 to obtain a compound of the following Chemical Formula 6'; and
3') a coupling reaction of the compound of the following Chemical Formula 6" obtained in step 2") with a compound of the following Chemical Formula 7, followed by condensation and hydrolysis to obtain a compound of the following Chemical Formula 1".

The base, ligand, conditions of the hydrolysis reaction, and the like used in the preparation method may all be applied as described in the preparation method of Compound 1.

This may be represented by Scheme 2 below.

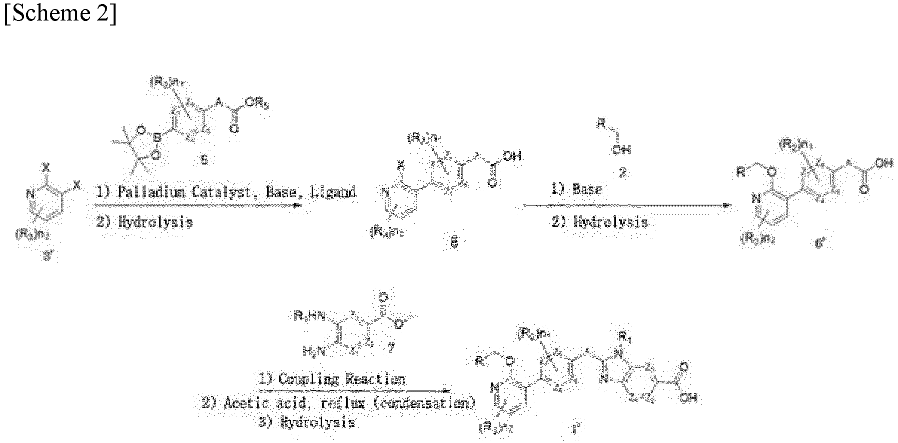

In a specific embodiment, when A is carbon in the compound of Chemical Formula 1" according to the present disclosure, the compound of Chemical Formula 1" may be prepared by a preparation method, comprising:
1") reacting a compound of the following Chemical Formula 2' with a compound of the following Chemical Formula 3' to obtain a compound of the following Chemical Formula 4';
2") reacting the compound of the following Chemical Formula 4' obtained in step 1") with a compound of the following Chemical Formula 5 under a palladium catalyst and then hydrolyzing to obtain a compound of the following Chemical Formula 6"; and
3") a coupling reaction of the compound of the following Chemical Formula 6" obtained in step 2") with a compound of the following Chemical Formula 7, followed by condensation and hydrolysis to obtain a compound of the following Chemical Formula 1".

The base, ligand, conditions of the hydrolysis reaction, and the like used in the preparation method may all be applied as described in the preparation method of Compound 1".

This may be represented by Scheme 3 below.

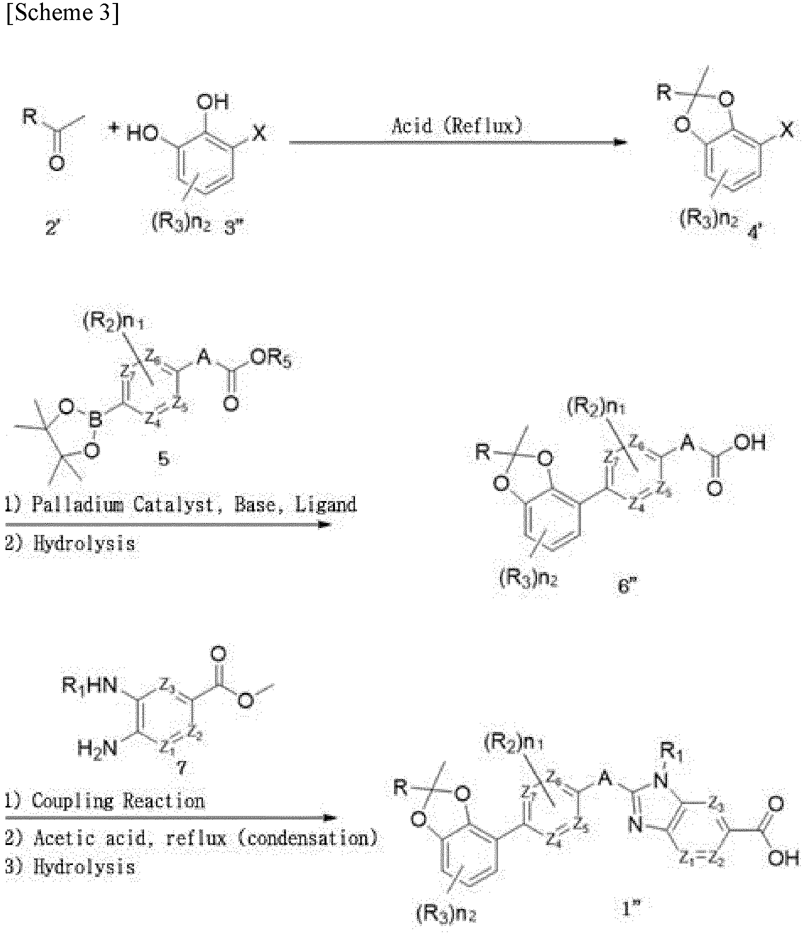

In Schemes 1 to 3, m, n₁, n₂, n₃, Rₐ, R, R₁, R₂, R₃, R₄, Z₁, Z₂, Z₃, Z₄, Z₅, Z₆ and Z₇ are as defined in Chemical Formula 1 above;
R₅ is alkyl; and
X is halo, preferably Cl, Br or I.

A compound not specifically described in the preparation method of the present specification is a compound known per se, or a compound that may be easily synthesized from a known compound by a known synthesis method or a method similar thereto.

The compound of Chemical Formula 1, Chemical Formula 1' or Chemical Formula 1" obtained through the above method may be isolated or purified from the reaction product by various methods such as recrystallization, iontophoresis, silica gel column chromatography, or ion exchange resin chromatography.

As described above, the compound according to the present disclosure, a starting material or intermediate for its preparation, and the like may be synthesized by various methods, and these methods need to be interpreted as falling within the scope of the present disclosure with respect to the preparation of a compound represented by Chemical Formula 1, Chemical Formula 1' or Chemical Formula 1".

### [Effects of the present disclosure]

The new compound according to the present disclosure is useful as an agent for treating or preventing obesity or various metabolic diseases such as diabetes and hyperlipidemia through excellent GLP-1 agonist activity and excellent DMPK profile.

Hereinafter, the present disclosure will be described in detail by way of Examples. However, the following Examples and Experimental Examples are merely provided to illustrate the present disclosure, and are provided for easier understanding of the present disclosure, and the content of the present disclosure is not limited thereto.

In this example, the following abbreviations are defined to represent the following substances.
- DMF: Dimethyl formamide
- THF: Tetrahydrofuran
- TEA: Triethylamine
- EtOAc: Ethyl acetate
- MgSO₄: Magnesium sulfate
- MPLC: High-speed automatic separation and purification system
- Pd/C: Palladium/Carbon
- AcOH: Acetic acid
- HCl: Hydrochloric acid
- CS₂: Carbon disulfide
- NaH: Sodium hydride
- DCM: Dichloromethane
- mCPBA: 3-chloroperbenzoic acid
- NaHCO₃: Hydrocarbon sodium
- t-BuOK: Potassium tert-butoxide
- Cs2CO₃: Cesium carbonate
- K₂CO₃: Potassium carbonate
- BINAP: (2,2'-bis(diphenylphosphino)-1,1'-binaphthyl)
- Pd₂(dba)₃: Tris(dibenzylideneacetone)dipalladium
- Fe: Iron
- NH₄Cl: Ammonium Chloride
- CDI: Carbodiimidazole
- DCE: 1,2-Dichloroethane
- POCl₃: Phosphoryl chloride
- NaOH: Sodium hydroxide
- H₂O: Water
- MeOH: Methanol
- HOBt: Hydroxybenzotriazole
- EDC: 1-ethyl-3-(3-dimethylaminopropyl)carbodiimide
- Dppf: 1,1'-bis(diphenylphosphino)ferrocene
- Na₂SO₄: Sodium sulfate
- NMR: Nuclear magnetic resonance
- HATU: (1-[bis(dimethylamino)methylene]-1H-1,2,3-triazolo[4,5-b]pyridinium 3-oxide hexafluorophosphate

### [Preparation Example 1: Preparation of methyl (S)-4-nitro-3-((oxetan-2-ylmethyl)amino)benzoate]

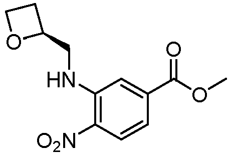

### Methyl (S)-4-nitro-3-((oxetan-2-ylmethyl)amino)benzoate

(S)-Oxytan-2-ylmethylamine (539 mg, 6.19 mmol) was dissolved in DMF (7 milliliter) and THF (~10 mL), then TEA (2.59 mL, 18.56 mmol) and methyl 3-fluoro-4-nitrobenzoate (1.23 g, 6.19 mmol) were added and stirred at room temperature under nitrogen for 16 h. After completion of the reaction, the reaction solvent was concentrated under reduced pressure. Water was added, extracted with EtOAc, then dried over MgSO₄ and concentrated under reduced pressure. Purification by MPLC gave methyl (,S)-4-nitro-3-((oxetan-2-ylmethyl)amino)benzoate (813 mg, 3.05 mmol, 49%).
¹H NMR (500 MHz, CDCl₃) δ 8.36 (br s, 1H), 8.24 (d, *J* = 8.5 Hz, 1H), 7.63 (d, *J =* 1.5 Hz, 1H), 7.28 (d, *J =* 1.5 Hz, 1H), 5.16-5.20 (m, 1H), 4.73-4.76 (m, 1H), 4.61-4.65 (m, 1H), 3.94 (s, 3H), 3.62-3.65 (m, 2H), 2.76-2.80 (m, 1H), 2.59-2.75 (m, 1H); LC-MS(ESI): 267.26 [M+H]⁺

### [Preparation Example 2: Preparation of methyl (S)-4-amino-3-((oxetan-2-ylmethyl)amino)benzoate]

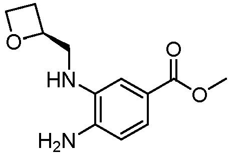

### Methyl (S)-4-amino-3-((oxetan-2-ylmethyl)amino)benzoate

Methyl (*S*)-4-nitro-3-((oxytan-2-ylmethyl)amino)benzoate (813 mg, 3.05 mmol) obtained in Preparation Example 1 was dissolved in THF (13 mL), then 10% Pd/C (325 mg) was added and stirred at room temperature under hydrogen for 4 h. After completion of the reaction, it was filtered with EtOAc through celite, and then concentrated under reduced pressure to obtain methyl (*S*)-4-amino-3-((oxetan-2-ylmethyl)amino)benzoate.
¹H NMR (500 MHz, CDCl₃) δ 7.48 (dd, *J =* 8.0, 1.5 Hz, 1H), 7.38 (d, *J =* 1.5 Hz, 1H), 6.68 (d, *J =* 8.0 Hz, 1H), 5.12-5.10 (m, 1H), 4.77-4.73 (m, 1H), 4.64-4.59 (m, 1H), 3.86 (s, 5H), 3.53 (br s, 1H), 3.46-3.42 (m, 1H), 3.37-3.34 (m, 1H), 2.78-2.74 (m, 1H), 2.61-2.57 (m, 1H); LC-MS(ESI): 237.27 [M+H]⁺

### [Preparation Example 3: Preparation of methyl 3-(((1-ethyl-1H-imidazol-5-yl)methyl)amino)-4-nitrobenzoate]

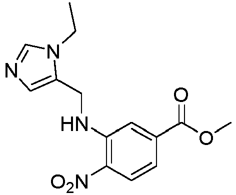

### Methyl 3-(((1-ethyl-1H-imidazol-5-yl)methyl)amino)-4-nitrobenzoate

Methyl 3-fluoro-4-nitrobenzoate (700 mg, 3.52 mmol) was dissolved in THF (12 mL), then (1-ethyl-1*H*-imidazol-5-yl)methanamine dihydrochloride (696) mg, 3.52 mmol) and TEA (1.47 mL, 10.55 mmol) were added and stirred at 80 °C under nitrogen for 24 h. After completion of the reaction, it was cooled to room temperature, and then the reaction solvent is concentrated under reduced pressure. Water was added, extracted with EtOAc, then dried over MgSO₄ and concentrated under reduced pressure. MPLC gave methyl 3-(((1-ethyl-1*H-*imidazol-5-yl)methyl)amino)-4-nitrobenzoate (442 mg, 1.452 mmol, 41%).
¹H NMR (500 MHz, CDCl₃) δ 8.25 (d, *J* = 9.0 Hz, 1H), 7.94 (br s, 1H), 7.69 (d, *J =* 1.5 Hz, 1H), 7.57 (s, 1H), 7.34 (dd, *J =* 9.0, 1.5 Hz, 1H), 7.11 (s, 1H), 4.54 (d, *J =* 5.0 Hz, 2H), 3.99 (m, 5H), 1.48 (t, *J =* 7.5 Hz, 3H); LC-MS(ESI): 305.1 [M+H]⁺

### [Preparation Example 4: Preparation of methyl 4-amino-3-(((1-ethyl-1H-imidazol-5-yl)methyl)amino)benzoate]

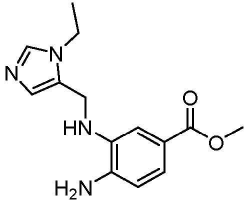

### Methyl 4-amino-3-(((1-ethyl-1H-imidazol-5-yl)methyl)amino)benzoate

Methyl 3-(((1-ethyl-1*H*-imidazol-5-yl)methyl)amino)-4-nitrobenzoate (440 mg, 1.446 mmol) obtained in Preparation Example 3 was dissolved in MeOH/H₂O (6 mL/2 mL), then Fe (242 mg, 4.34 mmol) and NH₄Cl (1.55 g, 28.9 mmol) were added and stirred at 80 °C under nitrogen for 3 h. After completion of the reaction, it was cooled to room temperature, water was added, extracted with EtOAc, dried over MgSO₄, and concentrated under reduced pressure. MPLC gave methyl 4-amino-3-(((1-ethyl-1H-imidazol-5-yl)methyl)amino)benzoate (254 mg, 0.925 mmol, 64%).
¹H NMR (400 MHz, CDCl₃) δ 7.53-7.50 (m, 2H), 7.46 (d, *J =* 1.6 Hz, 1H), 7.03 (s, 1H), 6.70 (d, *J* = 8.4 Hz, 1H), 4.27 (s, 2H), 4.01 (q, *J =* 7.4 Hz, 2H), 3.87 (s, 3H), 1.45 (t, *J =* 7.2 Hz, 3H); LC-MS(ESI): 275.1 [M+H]⁺

### [Preparation Example 5: Preparation of methyl 4-nitro-3-((oxazol-5-ylmethyl)amino)benzoate]

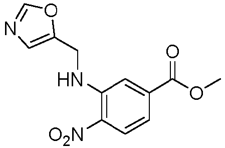

### Methyl 4-nitro-3-((oxazol-5-ylmethyl)amino)benzoate

Methyl 3-fluoro-4-nitrobenzoate (904 mg, 4.54 mmol) was dissolved in THF (15 mL), then oxazol-5-ylmethanamine hydrochloride (600 mg, 4.33 mmol) and TEA (1.81 mL, 12.98 mmol) were added and stirred at room temperature under nitrogen for 16 h. After completion of the reaction, the reaction solvent was concentrated under reduced pressure. Water was added, extracted with EtOAc, then dried over MgSO₄ and concentrated under reduced pressure. Purification by MPLC gave methyl 4-nitro-3-((oxazol-5-ylmethyl)amino)benzoate (682 mg, 2.46 mmol, 57%).
¹H NMR(400 MHz, CDCl₃) δ 8.25-8.23 (m, 2H), 7.87 (s, 1H), 7.64 (d*, J =* 1.2 Hz, 1H), 7.33 (dd, *J = 2.0,* 1.2 Hz, 1H), 7.09 (s, 1H), 4.66 (d, *J =* 5.6 Hz, 2H), 3.94 (s, 3H)

### [Preparation Example 6: Preparation of methyl 4-amino-3-((oxazol-5-ylmethyl)amino)benzoate]

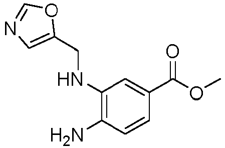

### Methyl 4-amino-3-((oxazol-5-ylmethyl)amino)benzoate

Methyl 4-nitro-3-((oxazol-5-ylmethyl)amino)benzoate (682 mg, 2.46 mmol) obtained in Preparation Example 5 was dissolved in THF (12 mL), and then 10% Pd/C (262 mg) was added and stirred at room temperature under hydrogen for 24 h. After completion of the reaction, it was filtered with EtOAc through celite, and then concentrated under reduced pressure to obtain methyl 4-amino-3-((oxazol-5-ylmethyl)amino)benzoate (600 mg, 2.43 mmol, 99%).
¹H NMR (500 MHz, CDCl₃) δ 7.86 (s, 1H), 7.52 (dd, *J =* 8.0, 1.5 Hz, 1H), 7.43 (d, *J* = 2.0 Hz, 1H), 7.04 (s, 1H), 6.72 (d, *J=* 8.0 Hz, 1H), 4.41 (d, *J =* 5.5 Hz, 2H), 3.86 (s, 3H), 3.83 (s, 2H), 3.46 (s,1H); LC-MS(ESI): 248.25 [M+H]⁺

### [Preparation Example 7: Preparation of methyl (S)-4-nitro-3-(((tetrahydrofuran-2-yl)methyl)amino)benzoate]

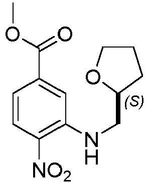

### Methyl (S)-4-nitro-3-(((tetrahydrofuran-2-yl)methyl)amino)benzoate

Methyl 3-fluoro-4-nitrobenzoate (1.39 g, 7.0 mmol) was dissolved in DMF (2.5 mL), then (S)-(tetrahydrofuran-2-yl)methanamine (0.868 mL) and K₂CO₃ (967 mg) were added at room temperature. The reactant was stirred at 50 °C for 4 h. Water was added, then, it was extracted with EtOAc. The organic layer was dried over Na₂SO₄, concentrated under reduced pressure, and purified by MPLC to obtain methyl (S)-4-nitro-3-(((tetrahydrofuran-2-yl)methyl)amino)benzoate (1.69 g, 6.86 mmol, 86%).
¹H NMR (400 MHz, CDCl₃) δ 8.26 - 8.12 (m, 2H), 7.59 (d, *J =* 1.8 Hz, 1H), 7.24 (dd, *J =* 9.1, 1.8 Hz, 1H), 4.22 (qd, *J =* 6.9, 4.1 Hz, 1H), 4.02 - 3.91 (m, 4H), 3.87 - 3.77 (m, 1H), 3.53 (td, *J =* 8.7, 4.3 Hz, 1H), 3.46 - 3.34 (m, 1H), 2.18 - 2.04 (m, 1H), 2.05 - 1.90 (m, 2H), 1.82 - 1.61 (m, 1H); LC-MS(ESI): 281.28 [M+H]⁺

### [Preparation Example 8: Preparation of methyl (S)-4-amino-3-(((tetrahydrofuran-2-yl)methyl)amino)benzoate]

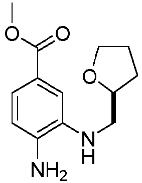

### Methyl (S)-4-amino-3-(((tetrahydrofuran-2-yl)methyl)amino)benzoate

Methyl (S)-4-nitro-3(((tetrahydrofuran-2-yl)methyl)amino)benzoate (1.69 g, 6.86 mmol) obtained in Preparation Example 7 was dissolved in THF (50 mL), then 10% Pd/C (169 mg) was added and stirred at room temperature under hydrogen for 17.5 h. After completion of the reaction, Pd/C was filtered through celite and concentrated under reduced pressure to obtain methyl (S)-4-amino-3-(((tetrahydrofuran-2-yl)methyl)amino)benzoate (1.68 g, 6.72 mmol, 98%).
¹H NMR (500 MHz, CDCl₃) δ 7.46 (dd, *J =* 8.1, 1.7 Hz, 1H), 7.34 (d, *J =* 1.5 Hz, 1H), 6.67 (d, *J =* 7.9 Hz, 1H), 4.20 (qd, *J =* 7.3, 3.2 Hz, 1H), 3.96 - 3.76 (m, 5H), 3.53 (brs, 1H), 3.26 (dd, *J =* 11.9, 3.1 Hz, 1H), 3.15 - 3.03 (m, 1H), 2.14 - 2.04 (m, 1H), 2.00 - 1.90 (m, 2H), 1.77 - 1.63 (m, 1H); LC-MS(ESI): 251.29 [M+H]⁺

### [Preparation Example 9: Preparation of 4-bromo-2-(4-chloro-2-fluorophenyl)-2-methylbenzo[d][1,3]dioxole]

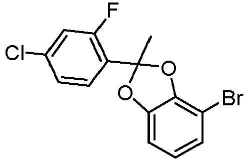

### 4-bromo-2-(4-chloro-2-fluorophenyl)-2-methylbenzo[d][1,3]dioxole

3-bromobenzen-1,2-diol (20 g, 105.814 mmol) was dissolved in toluene (211 mL), then 1-(4-chloro-2-fluorophenyl)ethan-1-one (19.08 g, 110.576 mmol) and PTSA-H₂O (402 mg, 2.116 mmol) were added and refluxed under a Dean-Stark trap for 96 h. It was concentrated under reduced pressure, toluene was removed, and purified by MPLC to obtain 4-bromo-2-(4-chloro-2-fluorophenyl)-2-methylbenzo[d][1,3]dioxole (9.163 g, 26.7 mmol, 25.2%).

¹H NMR (500 MHz, CDCl₃) δ 7.54 (t, *J =* 8.1 Hz, 1H), 7.12 - 7.17 (m, 2H), 6.95 (d, *J* = 7.9 Hz, 1H), 6.75 (d, *J =* 7.6 Hz, 1H), 6.70 (t, *J =* 7.9 Hz, 1H), 2.11 (s, 3H).

### [Preparation Example 10: Preparation of methyl 2-(4-(2-(4-chloro-2-fluorophenyl)-2-methylbenzo[d][1,3]dioxol-4-yl)phenyl)acetate]

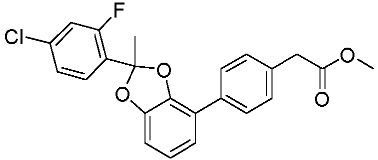

### Methyl 2-(4-(2-(4-chloro-2-fluorophenyl)-2-methylbenzo[d][1,3]dioxol-4-yl)phenyl)acetate

4-bromo-2-(4-chloro-2-fluorophenyl)-2-methylbenzo[d][1,3]dioxole (1.32 g, 3.85 mmol) obtained in Preparation Example 9, Pd(dppf)Cl₂-DCM (315 mg, 0.385 mmol), Cs2CO₃ (3.14 g, 9.63 mmol), and methyl 2-(4-(4,4,5,5-tetramethyl-1,3,2-dioxaborolan-2-yl)phenyl)acetate (1.08 g, 3.89 mmol) were dissolved in 17 mL of THF/H₂O (9/1) and substituted with nitrogen. The reactant was stirred at 85 °C for 24 h. After completion of the reaction, it was cooled to room temperature, filtered through celite using EtOAc, and concentrated under reduced pressure. MPLC gave methyl 2-(4-(2-(4-chloro-2-fluorophenyl)-2-methylbenzo[d][1,3]dioxol-4-yl)phenyl)acetate (1.2 g, 2.91 by MPLC) mmol, 75%).

¹H NMR (500 MHz, CDCl₃) δ 7.74 (d, *J =* 8.2 Hz, 2H), 7.55 (t, *J =* 8.2 Hz, 1H), 7.40 (d, *J =* 8.2 Hz, 2H), 7.15 (dd, *J =* 23.3, 10.5 Hz, 2H), 7.07 (d, *J =* 7.9 Hz, 1H), 6.92 (t, *J =* 7.8 Hz, 1H), 6.83 (d, *J =* 7.6 Hz, 1H), 3.75 (s, 3H), 3.70 (s, 2H), 2.12 (s, 3H).

### [Preparation Example 11: Preparation of 2-(4-(2-(4-chloro-2-fluorophenyl)-2-methylbenzo[d][1,3]dioxol-4-yl)phenyl)acetic acid]

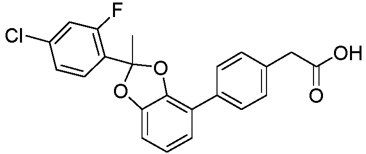

### 2-(4-(2-(4-chloro-2-fluorophenyl)-2-methylbenzo[d][1,3]dioxol-4-yl)phenyl)acetic acid

Methyl 2-(4-(2-(4-chloro-2-fluorophenyl)-2-methylbenzo[d][1,3]dioxol-4-yl)phenyl)acetate (1.17 g, 2.83 mmol) obtained in Preparation Example 10, 2.83 mmol) was dissolved in 10 mL of THF/H₂O (1/1), then NaOH (340 mg, 8.49 mmol) was added. The reactant was stirred at room temperature for 24 h. After completion of the reaction, the pH was adjusted to ~2 using 1N HCl, and then extracted with EtOAc. The organic layer was dried over Na₂SO₄, concentrated under reduced pressure, and purified by MPLC to obtain 2-(4-(2-(4-chloro-2-fluorophenyl)-2-methylbenzo[d][1,3]dioxol-4-yl)phenyl)acetic acid (1.10 g, 2.76 mmol, 97%).

¹H NMR (500 MHz, MeOD) δ 7.73-7.75 (m, 2H), 7.59 (t, *J =* 8.2 Hz, 1H), 7.39 (d, *J=* 7.9 Hz, 2H), 7.31 (dd, *J =* 10.8, 2.0 Hz, 1H), 7.22 (d, *J =* 8.5 Hz, 1H), 7.11 (d, *J =* 7.9 Hz, 1H), 6.94 (t, *J =* 7.8 Hz, 1H), 6.84 (d, *J =* 7.9 Hz, 1H), 3.66-3.70 (m, 2H), 2.10 (s, 3H).

### [Preparation Example 12: Preparation of methyl 2-(4-(2-(4-chloro-2-fluorophenyl)-2-methylbenzo[d][1,3]dioxol-4-yl)-3-fluorophenyl)acetate]

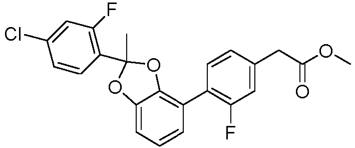

### Methyl 2-(4-(2-(4-chloro-2-fluorophenyl)-2-methylbenzo[d][1,3]dioxol-4-yl)-3-fluorophenyl)acetate

4-bromo-2-(4-chloro-2-fluorophenyl)-2-methylbenzo[d][1,3]dioxole (1.17 g, 3.40 mmol) obtained in Preparation Example 9, Pd(dppf)Cl₂-DCM (278 mg, 0.340 mmol), Cs2CO₃ (2.77 g, 8.50 mmol), and methyl 2-(4-(4,4,5,5-tetramethyl-1,3,2-dioxaborolan-2-yl)phenyl)acetate (1.00 g, 3.40 mmol) was dissolved in 17 mL of THF/H₂O (9/1) and substituted with nitrogen. The reactant was stirred at 85 °C for 24 h. After completion of the reaction, it was cooled to room temperature, filtered through celite using EtOAc, and concentrated under reduced pressure. MPLC gave methyl 2-(4-(2-(4-chloro-2-fluorophenyl)-2-methylbenzo[*d*][1,3]dioxol-4-yl)-3-fluorophenyl)acetate (920 mg, 2.14 mmol, 63%).

¹H NMR (500 MHz, MeOD) δ 7.74 (d, *J =* 8.2 Hz, 2H), 7.55 (t, *J =* 8.2 Hz, 1H), 7.40 (d, *J =* 8.2 Hz, 2H), 7.15 (dd, *J =* 23.3, 10.5 Hz, 2H), 7.07 (d, *J =* 7.9 Hz, 1H), 6.92 (t, *J =* 7.8 Hz, 1H), 6.83 (d, *J =* 7.6 Hz, 1H), 3.75 (s, 3H), 3.70 (s, 2H), 2.12 (s, 3H).

### [Preparation Example 13: Preparation of 2-(4-(2-(4-chloro-2-fluorophenyl)-2-methylbenzo[d][1,3]dioxol-4-yl)-3-fluorophenyl)acetic acid]

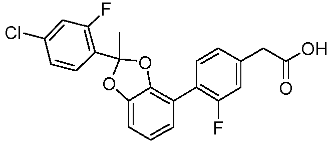

### 2-(4-(2-(4-chloro-2-fluorophenyl)-2-methylbenzo[d][1,3]dioxol-4-yl)-3-fluorophenyl)acetic acid

Methyl 2-(4-(2-(4-chloro-2-fluorophenyl)-2-methylbenzo[*d*][1,3]dioxol-4-yl)-3-fluorophenyl)acetate (920 mg, 2.135 mmol) obtained in Preparation Example 12 was dissolved in 14 mL of THF/H₂O (1/1), and then NaOH (214 mg, 5.34 mmol) was added. The reactant was stirred at room temperature for 24 h. After completion of the reaction, the pH was adjusted to ~2 using 1N HCl, and then extracted with EtOAc. The organic layer was dried over Na₂SO₄, concentrated under reduced pressure, and purified by MPLC to obtain 2-(4-(2-(4-chloro-2-fluorophenyl)-2-methylbenzo[*d*][1,3]dioxol-4-yl)-3-fluorophenyl)acetic acid (880 mg, 2.111 mmol, 99%).

¹H NMR (500 MHz, MeOD) δ 7.63 (t, *J =* 8.4 Hz, 1H), 7.42-7.48 (m, 1H), 7.30 (dd, *J* = 10.7, 1.8 Hz, 1H), 7.15-7.24 (m, 3H), 6.93-6.95 (m, 2H), 6.87-6.91 (m, 1H), 3.68 (d, *J =* 8.8 Hz, 2H), 2.04 (d, *J =* 10.1 Hz, 3H).

### [Preparation Example 14: Preparation of methyl 2-(4-(2-fluoropyridin-3-yl)phenyl)acetate]

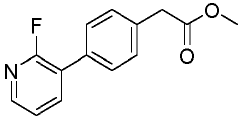

### methyl 2-(4-(2-fluoropyridin-3-yl)phenyl)acetate

3-bromo-2-fluoropyridine (2.00 g, 11.36 mmol), Pd(dppf)Cl₂-DCM (928 mg, 1.136 mmol), Cs2CO₃ (9.26 g, 28.4 mmol), and methyl 2-(4-(4),4,5,5-tetramethyl-1,3,2-dioxaborolan-2-yl)phenyl)acetate (3.45 g, 12.50 mmol) was dissolved in 34 mL of THF/H₂O (9/1), and substituted with nitrogen. The reactant was stirred at 85 °C for 24 h. After completion of the reaction, it was cooled to room temperature, filtered through celite using EtOAc, and concentrated under reduced pressure. MPLC gave methyl 2-(4-(2-fluoropyridin-3-yl)phenyl)acetate (2.70 g, 11.01 mmol, 97%).

¹H NMR (500 MHz, MeOD) δ 8.19 (t, *J =* 4.6 Hz, 1H), 8.04-8.08 (m, 1H), 7.57 (d, *J =* 8.2 Hz, 2H), 7.42 (d, *J=* 7.9 Hz, 3H), 3.74 (s, 2H), 3.72 (s, 3H).

### [Preparation Example 15: Preparation of 2-(4-(2-((4-chloro-2-fluorobenzyl)oxy)pyridin-3-yl)phenyl)acetic acid]

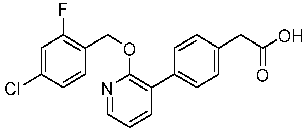

### 2-(4-(2-((4-chloro-2-fluorobenzyl)oxy)pyridin-3-yl)phenyl)acetic acid

(4-Chloro-2-fluorophenyl)methanol (655 mg, 4.08 mmol) was dissolved in 30 mL of DMF and NaH (60 % dispersion in mineral oil, 489 mg, 12.23 mmol) was added at 0 °C. After stirring at the same temperature for 10 min, methyl 2-(4-(2-fluoropyridin-3-yl)phenyl)acetate (1.00 g, 4.08 mmol) obtained in Preparation Example 14 was added, and then stirred at room temperature for 5 h. After completion of the reaction, water was added at 0 °C and extracted with EtOAc. The organic layer was dried over Na₂SO₄, then concentrated under reduced pressure, and purified by MPLC to obtain methyl 2-(4-(2-((4-chloro-2-fluorobenzyl)oxy)pyridin-3-yl)phenyl)acetate (890 mg, 2.307 mmol). Without further purification, it was dissolved in THF/H₂O (5 mL/5 mL), then NaOH (277 mg, 6.92 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave 2-(4-(2-((4-chloro-2-fluorobenzyl)oxy)pyridin-3-yl)phenyl)acetic acid (450 mg, 1.210 mmol, 53%).

¹H NMR (400 MHz, CDCl₃) δ 8.14 (q, *J =* 2.2 Hz, 1H), 7.63 (dd, *J =* 7.3, 1.8 Hz, 1H), 7.56-7.49 (m, 2H), 7.37-7.30 (m, 3H), 7.13-7.02 (m, 2H), 6.99 (dd, *J =* 7.3, 5.0 Hz, 1H), 5.46 (s, 2H), 3.70 (s, 2H).

### [Preparation Example 16: Preparation of methyl 4-nitro-3-(((4-propyl-4H-1,2,4-triazol-3-yl)methyl)amino)benzoate]

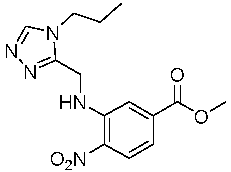

### Methyl 4-nitro-3-(((4-propyl-4H-1,2,4-triazol-3-yl)methyl)amino)benzoate

Methyl 3-fluoro-4-nitrobenzoate (934 mg, 4.69 mmol) was dissolved in THF (10 mL)/MeOH (6.67 mL), then (4-propyl-4H-1,2,4-triazol-3-yl)methanamine dihydrochloride (696 mg, 4.69 mmol) and TEA (3.27 mL, 23.46 mmol) were added and stirred under nitrogen at room temperature for 24 h. After completion of the reaction, it was cooled to room temperature, and then the reaction solvent is concentrated under reduced pressure. Water was added, extracted with EtOAc, then dried over MgSO₄ and concentrated under reduced pressure. MPLC gave methyl 4-nitro-3-(((4-propyl-4H-1,2,4-triazol-3-yl)methyl)amino)benzoate (918 mg, 2.87 mmol, 61%).

¹H NMR (500 MHz, MeOD) δ 8.54 (s, 1H), 8.27 (d, *J =* 8.8 Hz, 1H), 7.76 (d, *J =* 1.5 Hz, 1H), 7.34 (dd, *J =* 8.5, 1.5 Hz, 1H), 4.91 (d, *J =* 3.4 Hz, 2H), 4.15 (t, *J =* 7.5 Hz, 2H), 3.93 (s, 3H), 1.81-1.91 (m, 2H), 0.98 (t, *J =* 7.3 Hz, 3H).

### [Preparation Example 17: Preparation of methyl 4-amino-3-(((4-propyl-4H-1,2,4-triazol-3-yl)methyl)amino)benzoate]

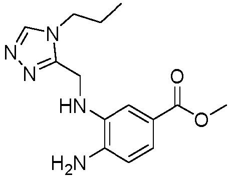

### Methyl 4-amino-3-(((4-propyl-4H-1,2,4-triazol-3-yl)methyl)amino)benzoate

Methyl 4-nitro-3-(((4-propyl-4*H*-1,2,4-triazol-3-yl)methyl)amino)benzoate (918 mg, 2.87 mmol) obtained in Preparation 16 was dissolved in THF (10 mL), then 10% Pd/C (922 mg) was added and stirred at room temperature under hydrogen for 24 h. After completion of the reaction, it was filtered through celite with EtOAc, and then concentrated under reduced pressure. MPLC gave methyl 4-amino-3-(((4-propyl-4H-1,2,4-triazol-3-yl)methyl)amino)benzoate (500 mg, 1.73 mmol, 60%).

¹H NMR (500 MHz, MeOD) δ 8.55 (d, *J=* 25.0 Hz, 1H), 7.39 (dd, *J = 8.1,* 1.7 Hz, 1H), 7.32-7.35 (m, 1H), 6.70 (d, *J =* 8.2 Hz, 1H), 4.62 (d, *J =* 18.3 Hz, 2H), 4.12 (t, *J =* 7.5 Hz, 2H), 3.80-3.88 (m, 3H), 1.82-1.91 (m, 2H), 0.92-1.02 (m, 3H).

### [Preparation Example 18: Preparation of bicyclo[2.2.2]octan-1-ylmethanol]

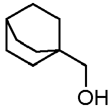

### Bicyclo[2.2.2]octan-1-ylmethanol

Bicyclo[2.2.2]octan-1-carboxylic acid (1110 mg, 7.198 mmol) was dissolved in Et₂O, and LiAH₄ (409 mg, 10.797 mmol) was slowly added at 0 °C. After stirred at room temperature for 1 h, the temperature was again lowered to 0 °C, and 0.41 mL of water was slowly added. 0.41 mL of 15% NaOH (aq) was added, an additional 1.23 mL of water was added, and then stirred at room temperature for 15 min. The aluminum complex was filtered through filter paper and then concentrated under reduced pressure to obtain bicyclo[2.2.2]octan-1-ylmethanol (972.8 mg, 6.937 mmol, 96%).

¹H NMR (500 MHz, CDCl₃) δ 3.21 (d, *J =* 5.8 Hz, 2H), 1.34 - 1.61 (m, 13H).

### [Preparation Example 19: Preparation of 2-(bicyclo[2.2.2]octan-1-ylmethoxy)-6-chloropyridine]

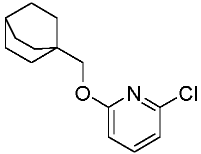

### 2-(bicyclo[2.2.2]octan-1-ylmethoxy)-6-chloropyridine

Bicyclo[2.2.2]octan-1-ylmethanol (537 mg, 3.63 mmol) obtained in Preparation Example 18 was dissolved in 10 mL of THF, and *t*-BuOK (733 mg, 6.534 mmol) was added at 0 °C. After stirring at the same temperature for 10 min, 2,6-dichloropyridine was added, and then stirred at room temperature for 1 h. After completion of the reaction, water was added, and extracted with EtOAc. The organic layer was dried over Na₂SO₄, concentrated under reduced pressure, and purified by MPLC to obtain 2-(bicyclo[2.2.2]octan-1-ylmethoxy)-6-chloropyridine (886 mg, 3.521 mmol, 97%).

¹H NMR (500 MHz, CDCl₃) δ 7.48 (t, *J =* 7.9 Hz, 1H), 6.85 (d, *J =* 7.6 Hz, 1H), 6.62 (d, *J = 8.2* Hz, 1H), 3.88 (s, 2H), 1.47 - 1.60 (m, 13H).

### [Preparation Example 20: Preparation of methyl 2-(4-(6-(bicyclo[2.2.2]octan-1-ylmethoxy)pyridin-2-yl)phenyl)acetate]

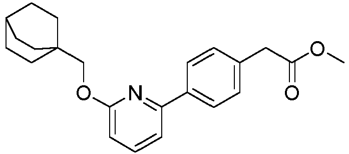

### Methyl 2-(4-(6-(bicyclo[2.2.2]octan-1-ylmethoxy)pyridin-2-yl)phenyl)acetate

2-(bicyclo[2.2.2]octan-1-ylmethoxy)-6-chloropyridine (541 mg, 2.149 mmol) obtained in Preparation Example 19 was dissolved in THF/H₂O (20 mL/2 mL), methyl 2-(4-(4,4,5,5-tetramethyl-1,3,2-dioxabororen-2-yl)phenyl)acetate (2967 mg, 10.74 mmol) and Cs2CO3 (1960 mg, 6.02 mmol)) was added and then substituted with nitrogen 3 times. Pd(dppf)Cl₂-DCM (87.7 mg, 0.107 mmol) was added, then substituted with nitrogen once more and stirred at 85 °C for 21 h. After completion of the reaction, it was cooled to room temperature, filtered through celite with EtOAc, and concentrated under reduced pressure. MPLC gave methyl 2-(4-(6-(bicyclo[2.2.2]octan-1-ylmethoxy)pyridin-2-yl)phenyl)acetate (415.9 mg, 1.138 mmol, 53%).

¹H NMR (500 MHz, CDCl₃) δ 7.97 (d, *J =* 8.2 Hz, 2H), 7.59 (t, *J =* 7.8 Hz, 1H), 7.37 (d, *J = 7.9* Hz, 2H), 7.27 (d, *J =* 7.6 Hz, 1H), 6.66 (d, *J =* 8.2 Hz, 1H), 4.03 (s, 2H), 3.71 (s, 3H), 3.68 (s, 2H), 1.52 - 1.62 (m, 13H).

### [Preparation Example 21: Preparation of 2-(4-(6-(bicyclo[2.2.2]octan-1-ylmethoxy)pyridin-2-yl)phenyl)acetic acid]

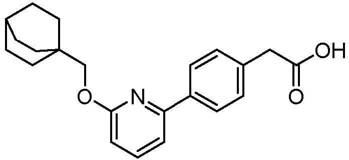

### 2-(4-(6-(bicyclo[2.2.2]octan-1-ylmethoxy)pyridin-2-yl)phenyl)acetic acid

Methyl 2-(4-(6-(bicyclo[2.2.2]octan-1-ylmethoxy)pyridin-2-yl)phenyl)acetate (415 mg, 1.138 mmol) obtained in Preparation 20 was dissolved in 6 mL of THF, and 2.85 mL of 1N NaOH was added. The reactant was stirred at room temperature for 19 h. After completion of the reaction, the pH was adjusted to 4 using 1N HCl, and then extracted with EtOAc. The organic layer was dried over Na₂SO₄, then concentrated under reduced pressure, and purified by MPLC to obtain 2-(4-(6-(bicyclo[2.2.2]octan-1-ylmethoxy)pyridin-2-yl)phenyl)acetic acid (376 mg, 1.070 mmol, 94%).

¹H NMR (500 MHz, CDCl₃) δ 7.98 (d, *J =* 8.2 Hz, 2H), 7.59 (t, *J =* 7.8 Hz, 1H), 7.37 (d, *J =* 8.2 Hz, 2H), 7.27 (d, *J =* 3.2 Hz, 1H), 6.67 (d, *J =* 8.2 Hz, 1H), 4.03 (s, 2H), 3.69 (s, 2H), 1.52 - 1.63 (m, 13H).

### [Preparation Example 22: Preparation of (S)-2-(4-(6-(bicyclo[2.2.2]octan-1-ylmethoxy)pyridin-2-yl)benzyl)-1-(oxetan-2-ylmethyl)-1H-benzo[d]imidazol-6-carboxylate]

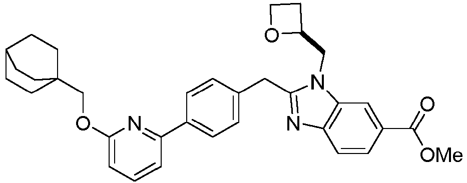

### Methyl (S)-2-(4-(6-(bicyclo[2.2.2]octan-1-ylmethoxy)pyridin-2-yl)benzyl)-1-(oxetan-2-ylmethyl)-1H-benzo[d]imidazol-6-carboxylate

Methyl methyl (*S*)-4-amino-3-((oxytan-2-ylmethyl)amino)benzoate (83 mg, 0.35 mmol) obtained in Preparation Example 2 and 2-(4-(6-(bicyclo[2.2.2]octan-1-ylmethoxy)pyridin-2-yl)phenyl)acetic acid (147 mg, 0.42 mmol) obtained in Preparation Example 21 were dissolved in 5 mL of DMF, then EDC (135 mg, 0.703 mmol) and HOBt (108 mg, 0.703 mmol) was added and stirred at room temperature for 15 h. After completion of the reaction, water was added, and extracted with EtOAc. The organic layer was dried over Na₂SO₄, concentrated under reduced pressure, and purified by MPLC to obtain the intermediate methyl (*S*)-4-(2-(4-(6-(bicyclo[2.2.2]octan-1-ylmethoxy)pyridin-2-yl)phenyl)acetamido)-3-((oxytan-2-ylmethyl)amino)benzoate. Without further purification, it was dissolved in 5 mL of acetic acid and stirred at 120 °C for 2 h. Water was added and extracted with EtOAc. The organic layer was dried over Na₂SO₄, concentrated under reduced pressure, and purified by MPLC to obtain methyl (*S*)-2-(4-(6-(bicyclo[2.2.2]octan-1-ylmethoxy)pyridin-2-yl)benzyl Obtained)-1-(oxytan-2-ylmethyl)-1*H*-benzo[*d*]imidazol-6-carboxylate (110 mg, 0.199 mmol, 57%).

¹H NMR (500 MHz, CDCl₃) δ 8.07 (s, 1H), 7.95 - 7.99 (m, 3H), 7.79 (d, *J =* 8.5 Hz, 1H), 7.58 (t, *J =* 7.8 Hz, 1H), 7.32 (d, *J =* 8.2 Hz, 2H), 7.25 (d, *J =* 7.3 Hz, 1H), 6.65 (d, *J =* 8.2 Hz, 1H), 5.07 (qd, *J =* 6.8, 2.7 Hz, 1H), 4.49 - 4.63 (m, 3H), 4.31 - 4.39 (m, 2H), 4.24 (dd, *J =* 15.6, 3.1 Hz, 1H), 4.00 (s, 2H), 3.94 (s, 3H), 2.61 - 2.68 (m, 1H), 2.29 - 2.36 (m, 1H), 1.49 - 1.64 (m, 13H).

### [Example 1: Preparation of (S)-2-(4-(2-((4-chloro-2-fluorobenzyl)oxy)pyridin-3-yl)benzyl)-1-(oxetan-2-ylmethyl)-1H-benzo[d]imidazol-6-carboxylic acid]

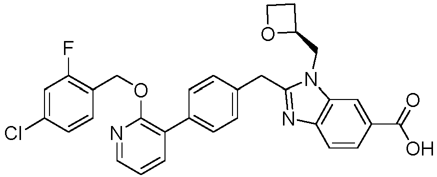

### (S)-2-(4-(2-((4-chloro-2-fluorobenzyl)oxy)pyridin-3-yl)benzyl)-1-(oxetan-2-ylmethyl)-1H-benzo[d]imidazol-6-carboxylic acid

2-(4-(2-((4-chloro-2-fluorobenzyl)oxy)pyridin-3-yl)phenyl)acetic acid (150 mg, 0.403 mmol) obtained in Preparation Example 15 was dissolved in DMF (2 mL), and then, methyl (*S*)-4-amino-3-((oxytan-2-ylmethyl)amino)benzoate (95 mg, 0.403 mmol) obtained in Preparation Example 2, EDC (155 mg, 0.807 mmol) and HOBt (124 mg, 0.807 mmol) were added. The reactant was stirred at room temperature under nitrogen for 24 h. After completion of the reaction, water was added, extracted with EtOAc, dried over MgSO₄, and concentrated under reduced pressure to obtain the intermediate methyl (*S*)-4-(2-(4-(2-((4-chloro-2-fluorobenzyl)pyridin-3-yl)phenyl)acetamido)-3-((oxytan-2-ylmethyl)amino)benzoate (210 mg, 0.356 mmol). Without further purification, it was dissolved in AcOH (2.04 mL, 35.6 mmol) and stirred at 120 °C for 3 h. After completion of the reaction, it was concentrated under reduced pressure. Water was added, extracted with EtOAc, then dried over MgSO₄ and concented under reduced pressure to obtain the intermediate methyl (*S*)-2-(4-(2-((4-chloro-2-fluorobenzyl)oxy)pyridin-3-yl)benzyl)-1-(oxytan-2-ylmethyl)-1*H*-benzo[*d*]imidazol-6-carboxylate (131 mg, 0.229 mmol). Without further purification, it was dissolved in THF/H₂O (1 mL/1 mL), then NaOH (28 mg, 0.687 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave (*S*)-2-(4-(2-((4-chloro-2-fluorobenzyl)oxy)pyridin-3-yl)benzyl)-1-(oxytan-2-ylmethyl)-1*H*-benzo[d]imidazol-6-carboxylic acid (50 mg, 0.090 mmol, 39%).

¹H NMR (500 MHz, MeOD) δ 8.30 (s, 1H), 8.15 (dd, *J=* 4.9, 1.5 Hz, 1H), 7.99 (d, *J=* 8.5 Hz, 1H), 7.74 (dd, *J =* 7.3, 1.8 Hz, 1H), 7.70 (d, *J =* 8.5 Hz, 1H), 7.55 (d, *J =* 8.2 Hz, 2H), 7.41 (t, *J =* 8.1 Hz, 1H), 7.33 (d, *J =* 8.2 Hz, 2H), 7.21 (dd, *J =* 10.1, 1.8 Hz, 1H), 7.13 (d, *J =* 8.2 Hz, 1H), 7.08 (dd, *J =* 7.3, 4.9 Hz, 1H), 5.45 (s, 2H), 4.96-5.00 (m, 1H), 4.53-4.62 (m, 2H), 4.51 (s, 2H), 4.45 (dd, *J =* 15.6, 2.4 Hz, 1H), 4.37-4.41 (m, 1H), 2.59-2.63 (m, 1H), 2.32-2.36 (m, 1H).

### [Example 2: Preparation of 2-(4-(2-(4-chloro-2-fluorophenyl)-2-methylbenzo[d][1,3]dioxol-4-yl)benzyl)-1-(((S)-oxetan-2-yl)methyl)-1H-benzo[d]imidazol-6-carboxylic acid]

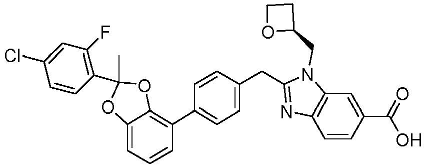

### 2-(4-(2-(4-chloro-2-fluorophenyl)-2-methylbenzo[d][1,3]dioxol-4-yl)benzyl)-1-(((S)-oxetan-2-yl)methyl)-1H-benzo[d]imidazol-6-carboxylic acid

2-(4-(2-(4-chloro-2-fluorophenyl)-2-methylbenzo[*d*][1,3]dioxol-4-yl)phenyl)acetic acid (150 mg) obtained in Preparation Example 11, 0.376 mmol) was dissolved in DMF (3 mL), and then methyl (*S*)-4-amino-3-((oxytan-2-ylmethyl)amino)benzoate (107 mg, 0.451 mmol) obtained in Preparation Example 2, EDC (144 mg, 0.752 mmol), HOBt (115 mg, 0.752 mmol) were added. The reactant was stirred at room temperature under nitrogen for 24 h. After completion of the reaction, water was added, extracted with EtOAc, dried over MgSO₄, and concentrated under reduced pressure to obtain the intermediate methyl 4-(2-(4-(2-(4-chloro-2-fluorophenyl)-2-methylbenzo[*d*][1,3]dioxol-4-yl)phenyl)acetamido)-3-((((*S*)-oxytan-2-yl)methyl)amino)benzoate (170 mg, 0.275 mmol). Without further purification, it was dissolved in AcOH (1.58 mL, 27.5 mmol) and stirred at 120 °C for 3 h. After completion of the reaction, it was concentrated under reduced pressure. Water was added, extracted with EtOAc, then dried over MgSO₄ and concented under reduced pressure to obtain the intermediate methyl 2-(4-(2-(4-chloro-2-fluorophenyl)-2-methylbenzo[*d*][1,3]dioxol-4-yl)benzyl)-1-(((*S*)-oxytan-2-yl)methyl)-1*H*-benzo[*d*]imidazol-6-carboxylate (130 mg, 0.217 mmol). Without further purification, it was dissolved in THF/H₂O (1 mL/1 mL), then NaOH (26 mg, 0.651 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave 2-(4-(2-(4-chloro-2-fluorophenyl)-2-methylbenzo[*d*][1,3]dioxol-4-yl)benzyl)-1-(((*S*)-oxytan-2-yl)methyl)-1H-benzo[d]imidazol-6-carboxylic acid (80 mg, 0.137 mmol, 63%).

¹H NMR (500 MHz, MeOD) δ 8.30 (s, 1H), 8.00 (dd, *J =* 8.5, 1.5 Hz, 1H), 7.76 (d, *J =* 8.2 Hz, 2H), 7.71 (d, *J =* 8.5 Hz, 1H), 7.57-7.63 (m, 1H), 7.40 (dd, *J=* 23.7, 8.1 Hz, 2H), 7.30 (d, *J =* 10.7 Hz, 1H), 7.22 (d, *J =* 8.5 Hz, 1H), 7.10 (d, *J =* 7.3 Hz, 1H), 6.93 (t, *J =* 7.9 Hz, 1H), 6.85 (d, *J =* 6.7 Hz, 1H), 5.05 (t, *J =* 7.2 Hz, 1H), 4.58-4.65 (m, 2H), 4.54 (s, 2H), 4.42-4.50 (m, 2H), 2.66-2.74 (m, 1H), 2.39-2.46 (m, 1H), 2.06 (d, *J =* 26.2 Hz, 3H).

### [Example 3: Preparation of 2-(4-(2-(4-chloro-2-fluorophenyl)-2-methylbenzo[d][1,3]dioxol-4-yl)-3-fluorobenzyl)-1-(((S)-oxetan-2-yl)methyl)-1H-benzo[d]imidazol-6-carboxylic acid]

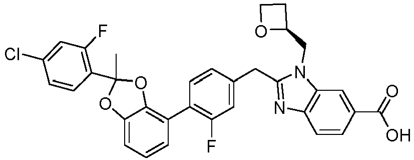

### 2-(4-(2-(4-chloro-2-fluorophenyl)-2-methylbenzo[d][1,3]dioxol-4-yl)-3-fluorobenzyl)-1-(((S)-oxetan-2-yl)methyl)-1H-benzo[d]imidazol-6-carboxylic acid

2-(4-(2-(4-chloro-2-fluorophenyl)-2-methylbenzo[*d*][1,3]dioxol-4-yl)-3-fluorophenyl)acetic acid (160 mg, 0.384 mmol) obtained in Preparation Example 13 was dissolved in DMF (2 mL), and then methyl (S)-4-amino-3-((oxytan-2-ylmethyl)amino)benzoate (91 mg, 0.384 mmol) obtained in Preparation Example 2, EDC (147 mg, 0.768 mmol), and HOBt (118 mg, 0.768 mmol) were added. The reactant was stirred at room temperature under nitrogen for 24 h. After completion of the reaction, water was added, extracted with EtOAc, dried over MgSO₄, and concentrated under reduced pressure to obtain the intermediate methyl 4-(2-(4-(2-(4-chloro-2-fluorophenyl)-2-methylbenzo[d][1,3]dioxol-4-yl)-3-fluorophenyl)acetamido)-3-((((*S*)-oxytan-2-yl)methyl)amino)benzoate (180 mg, 0.283 mmol). Without further purification, it was dissolved in AcOH (1.62 mL, 28.3 mmol) and stirred at 120 °C for 3 h. After completion of the reaction, it was concentrated under reduced pressure. Water was added, extracted with EtOAc, then dried over MgSO₄ and concented under reduced pressure to obtain the intermediate methyl 2-(4-(2-(4-chloro-2-fluorophenyl)-2-methylbenzo[*d*][1,3]dioxol-4-yl)-3-fluorobenzyl)-1-(((*S*)-oxytan-2-yl)methyl)-1*H-*benzo[*d*]imidazol-6-carboxylate (135 mg, 0.219 mmol). Without further purification, it was dissolved in THF/H₂O (1 mL/1 mL), then NaOH (26 mg, 0.656 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave 2-(4-(2-(4-chloro-2-fluorophenyl)-2-methylbenzo[*d*][1,3]dioxol-4-yl)-3-fluorobenzyl)-1-(((*S*)-oxytan-2-yl)methyl)-1*H-*benzo[d]imidazol-6-carboxylic acid (56 mg, 0.093 mmol, 42%).

¹H NMR (500 MHz, MeOD) δ 8.30 (s, 1H), 8.00 (d, *J =* 8.5 Hz, 1H), 7.70 (d, *J =* 8.5 Hz, 1H), 7.61 (t, *J =* 8.2 Hz, 1H), 7.48 (dq, *J =* 28.0, 7.6 Hz, 1H), 7.29 (dd, *J =* 11.0, 1.8 Hz, 1H), 7.15-7.23 (m, 3H), 6.87-6.93 (m, 3H), 5.10 (q, *J =* 6.4 Hz, 1H), 4.60-4.69 (m, 2H), 4.50-4.56 (m, 3H), 4.42-4.46 (m, 1H), 2.71-2.77 (m, 1H), 2.42-2.49 (m, 1H).

### [Example 4: Preparation of 2-(4-(2-(4-chloro-2-fluorophenyl)-2-methylbenzo[d][1,3]dioxol-4-yl)benzyl)-1-(oxazol-5-ylmethyl)-1H-benzo[d]imidazol-6-carboxylic acid]

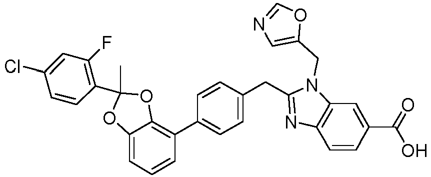

### 2-(4-(2-(4-chloro-2-fluorophenyl)-2-methylbenzo[d][1,3]dioxol-4-yl)benzyl)-1-(oxazol-5-ylmethyl)-1H-benzo[d]imidazol-6-carboxylic acid

2-(4-(2-(4-chloro-2-fluorophenyl)-2-methylbenzo[*d*][1,3]dioxol-4-yl)phenyl)acetic acid (150 mg, 0.376 mmol) obtained in Preparation Example 11 was dissolved in DMF (2 mL), and then methyl 4-amino-3-((oxazol--5-ylmethyl)amino)benzoate (102 mg, 0.414 mmol) obtained in Preparation Example 6, EDC (144 mg, 0.752 mmol), HOBt (115 mg, 0.752 mmol) were added. The reactant was stirred at room temperature under nitrogen for 24 h. After completion of the reaction, water was added, extracted with EtOAc, dried over MgSO₄, and concentrated under reduced pressure to obtain the intermediate methyl 4-(2-(4-(2-(4-chloro-2-fluorophenyl)-2-methylbenzo[*d*][1,3]dioxol-4-yl)phenyl)acetamido)-3-((oxazol-5-ylmethyl)amino)benzoate (139 mg, 0.221 mmol). Without further purification, it was dissolved in AcOH (1.27 mL, 22.13 mmol) and stirred at 120 °C for 3 h. After completion of the reaction, it was concentrated under reduced pressure. Water was added, extracted with EtOAc, then dried over MgSO₄ and concented under reduced pressure to obtain the intermediate methyl 2-(4-(2-(4-chloro-2-fluorophenyl)-2-methylbenzo[*d*][1,3]dioxol-4-yl)benzyl)-1-(oxazol-5-ylmethyl)-1*H*-benzo[d]imidazol-6-carboxylate (100 mg, 0.164 mmol). Without further purification, it was dissolved in THF/H₂O (1 mL/1 mL), then NaOH (20 mg, 0.492 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave 2-(4-(2-(4-chloro-2-fluorophenyl)-2-methylbenzo[*d*][1,3]dioxol-4-yl)benzyl)-1-(oxazol-5-ylmethyl)-1*H-*benzo[*d*]imidazol-6-carboxylic acid (20 mg, 0.034 mmol, 21%).

¹H NMR (500 MHz, MeOD) δ 8.31 (s, 1H), 8.01-8.05 (m, 2H), 7.71-7.74 (m, 3H), 7.59 (t, *J =* 8.2 Hz, 1H), 7.36 (d, *J =* 8.2 Hz, 2H), 7.30 (dd, *J =* 11.0, 1.8 Hz, 1H), 7.23 (d, *J =* 8.2 Hz, 1H), 7.10 (d, *J =* 7.9 Hz, 1H), 6.93 (t, *J =* 7.9 Hz, 2H), 6.85 (d, *J =* 7.6 Hz, 1H), 5.63 (s, 2H), 4.57 (d, *J =* 13.4 Hz, 2H), 2.03-2.14 (m, 3H).

### [Example 5: Preparation of 2-(4-(2-(4-chloro-2-fluorophenyl)-2-methylbenzo[d][1,3]dioxol-4-yl)-3-fluorobenzyl)-1-(oxazol-5-ylmethyl)-1H-benzo[d]imidazol-6-carboxylic acid]

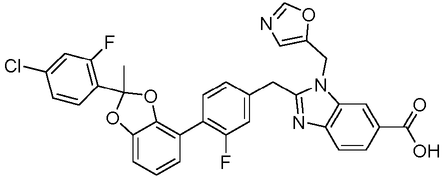

### 2-(4-(2-(4-chloro-2-fluorophenyl)-2-methylbenzo[d][1,3]dioxol-4-yl)-3-fluorobenzyl)-1-(oxazol-5-ylmethyl)-1H-benzo[d]imidazol-6-carboxylic acid

2-(4-(2-(4-chloro-2-fluorophenyl)-2-methylbenzo[*d*][1,3]dioxol-4-yl)-3-fluorophenyl)acetic acid (160 mg, 0.384 mmol) obtained in Preparation Example 13 was dissolved in DMF (2 mL), and then methyl 4-amino-3-((oxazol-5-ylmethyl)amino)benzoate (95 mg, 0.384 mmol) obtained in Preparation Example 6, EDC (147 mg, 0.768 mmol), and HOBt (118 mg, 0.768 mmol) were added. The reactant was stirred at room temperature under nitrogen for 24 h. After completion of the reaction, water was added, extracted with EtOAc, dried over MgSO₄, and concentrated under reduced pressure to obtain the intermediate methyl 4-(2-(4-(2-(4-chloro-2-fluorophenyl)-2-methylbenzo[*d*][1,3]dioxol-4-yl)-3-fluorophenyl)acetamido)-3-((oxazol-5-ylmethyl)amino)benzoate (148 mg, 0.229 mmol). Without further purification, it was dissolved in AcOH (1.31 mL, 22.9 mmol) and stirred at 120 °C for 3 h. After completion of the reaction, it was concentrated under reduced pressure. Water was added, extracted with EtOAc, then dried over MgSO₄ and concented under reduced pressure to obtain the intermediate methyl 2-(4-(2-(4-chloro-2-fluorophenyl)-2-methylbenzo[d][1,3]dioxol-4-yl)-3-fluorobenzyl)-1-(oxazol-5-ylmethyl)-1*H-*benzo[d]imidazol-6-carboxylate (100 mg, 0.159 mmol). Without further purification, it was dissolved in THF/H₂O (1 mL/1 mL), then NaOH (19 mg, 0.478 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave 2-(4-(2-(4-chloro-2-fluorophenyl)-2-methylbenzo[*d*][1,3]dioxol-4-yl)-3-fluorobenzyl)-1-(oxazol-5-ylmethyl)-1*H-*benzo[d]imidazol-6-carboxylic acid (27 mg, 0.044 mmol, 28%).

¹H NMR (500 MHz, MeOD) δ 8.32 (s, 1H), 8.05 (s, 1H), 8.02 (d, *J =* 8.5 Hz, 1H), 7.72 (d, *J =* 8.5 Hz, 1H), 7.60-7.65 (m, 1H), 7.47 (t, *J =* 7.9 Hz, 1H), 7.28-7.31 (m, 1H), 7.23 (d, *J* = 8.2 Hz, 1H), 7.14-7.17 (m, 2H), 7.05 (d, *J =* 9.2 Hz, 1H), 6.93 (d, *J =* 4.9 Hz, 2H), 6.89 (q, *J* = 4.4 Hz, 1H), 5.68 (s, 2H), 4.57 (s, 2H), 2.05 (s, 3H).

### [Example 6: Preparation of 2-(4-(2-((4-chloro-2-fluorobenzyl)oxy)pyridin-3-yl)benzyl)-1-(oxazol-5-ylmethyl)-1H-benzo[d]imidazol-6-carboxylic acid]

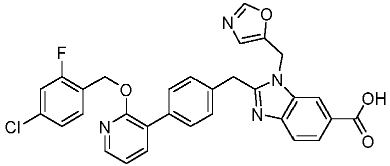

### 2-(4-(2-((4-chloro-2-fluorobenzyl)oxy)pyridin-3-yl)benzyl)-1-(oxazol-5-ylmethyl)-1H-benzo[d]imidazol-6-carboxylic acid

2-(4-(2-((4-chloro-2-fluorobenzyl)oxy)pyridin-3-yl)phenyl)acetic acid (110 mg, 0.296 mmol) obtained in Preparation Example 15 was dissolved in DMF (1.5 mL), and then, methyl 4-amino-3-((oxazol-5-ylmethyl)amino)benzoate (73 mg, 0.296 mmol) obtained in Preparation Example 6, EDC (113 mg, 0.592 mmol), and HOBt (91 mg, 0.592 mmol) were added. The reactant was stirred at room temperature under nitrogen for 24 h. After completion of the reaction, water was added, extracted with EtOAc, dried over MgSO₄, and concentrated under reduced pressure to obtain the intermediate methyl 4-(2-(4-(2-((4-chloro-2-fluorobenzyl)oxy)pyridin-3-yl)phenyl)acetamido)-3-((oxazol-5-ylmethyl)amino)benzoate (134 mg, 0.223 mmol). Without further purification, it was dissolved in AcOH (2.55 mL, 44.6 mmol) and stirred at 120 °C for 3 h. After completion of the reaction, it was concentrated under reduced pressure. Water was added, extracted with EtOAc, then dried over MgSO₄ and concented under reduced pressure to obtain the intermediate methyl 2-(4-(2-((4-chloro-2-fluorobenzyl)oxy)pyridin-3-yl)benzyl)-1-(oxazol-5-ylmethyl)-1*H*-benzo[d]imidazol-6-carboxylate (80 mg, 0.137 mmol). Without further purification, it was dissolved in THF/H₂O (1 mL/1 mL), then NaOH (16 mg, 0.412 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave 2-(4-(2-((4-chloro-2-fluorobenzyl)oxy)pyridin-3-yl)benzyl)-1-(oxazol-5-ylmethyl)-1*H*-benzo[*d*]imidazol-6-carboxylic acid (20 mg, 0.035 mmol, 26%).

¹H NMR (500 MHz, MeOD) δ 8.32 (s, 1H), 8.15 (d, *J = 3.1* Hz, 1H), 8.00-8.03 (m, 2H), 7.73 (t, *J =* 8.8 Hz, 2H), 7.53 (d, *J =* 8.2 Hz, 2H), 7.41 (t, *J =* 8.2 Hz, 1H), 7.31 (d, *J =* 8.2 Hz, 2H), 7.21 (d, *J =* 10.1 Hz, 1H), 7.07-7.12 (m, 2H), 6.86 (s, 1H), 5.62 (s, 2H), 5.45 (d, *J =* 6.4 Hz, 2H), 4.54 (s, 2H).

### [Example 7: Preparation of 2-(4-(2-((4-chloro-2-fluorobenzyl)oxy)pyridin-3-yl)benzyl)-1-((4-propyl-4H-1,2,4-triazol-3-yl)methyl)-1H-benzo[d]imidazol-6-carboxylic acid]

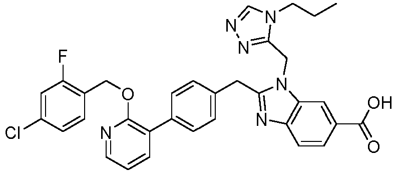

### 2-(4-(2-((4-chloro-2-fluorobenzyl)oxy)pyridin-3-yl)benzyl)-1-((4-propyl-4H-1,2,4-triazol-3-yl)methyl)-1H-benzo[d]imidazol-6-carboxylic acid

2-(4-(2-((4-chloro-2-fluorobenzyl)oxy)pyridin-3-yl)phenyl)acetic acid (150 mg, 0.403 mmol) obtained in Preparation Example 15 was dissolved in DMF (2 mL), and then, methyl 4-amino-3-(((4-propyl-4*H*-1,2,4-triazol-3-yl)methyl)amino)benzoate (117 mg, 0.403 mmol) obtained in Preparation Example 17, EDC (155 mg, 0.807 mmol), and HOBt (124 mg, 0.807 mmol) were added. The reactant was stirred at room temperature under nitrogen for 24 h. After completion of the reaction, water was added, extracted with EtOAc, dried over MgSO₄, and concentrated under reduced pressure to obtain the intermediate methyl 4-(2-(4-(2-((4-chloro-2-fluorobenzyl)oxy)pyridin-3-yl)phenyl)acetamido)-3-(((4-propyl-4*H*-1,2,4-triazol-3-yl)methyl)amino)benzoate (161 mg, 0.250 mmol). Without further purification, it was dissolved in AcOH (1.43 mL, 44.6 mmol) and stirred at 120 °C for 3 h. After completion of the reaction, it was concentrated under reduced pressure. Water was added, extracted with EtOAc, then dried over MgSO₄ and concented under reduced pressure to obtain the intermediate methyl 2-(4-(2-((4-chloro-2-fluorobenzyl)oxy)pyridin-3-yl)benzyl)-1-((4-propyl-4*H*-1,2,4-triazol-3-yl)methyl)-1*H*-benzo[*d*]imidazol-6-carboxylate (86 mg, 0.138 mmol). Without further purification, it was dissolved in THF/H₂O (1 mL/1 mL), then NaOH (17 mg, 0.413 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave 2-(4-(2-((4-chloro-2-fluorobenzyl)oxy)pyridin-3-yl)benzyl)-1-((4-propyl-4H-1,2,4-triazol-3-yl)methyl)-1*H-*benzo[*d*]imidazol-6-carboxylic acid (30 mg, 0.049 mmol, 36%).

¹H NMR (500 MHz, MeOD) δ 8.37 (s, 1H), 8.20 (s, 1H), 8.14 (dd, *J* = 4.9, 1.8 Hz, 1H), 8.03 (d, *J =* 8.2 Hz, 1H), 7.73-7.76 (m, 1H), 7.71 (dd, *J = 7.3,* 1.8 Hz, 1H), 7.48 (d, *J = 8.2* Hz, 2H), 7.44 (t, *J =* 7.9 Hz, 1H), 7.26 (d, *J =* 8.2 Hz, 2H), 7.23 (dd, *J* = 10.1, 1.8 Hz, 1H), 7.16 (d, *J =* 8.2 Hz, 1H), 7.08 (dd, *J =* 7.3, 4.9 Hz, 1H), 5.74-5.79 (m, 2H), 5.44 (d, *J =* 18.6 Hz, 2H), 4.46 (d, *J =* 10.4 Hz, 2H), 3.78 (t, *J =* 7.5 Hz, 2H), 1.47 (q, *J =* 7.4 Hz, 2H), 0.67-0.72 (m, 3H).

### [Example 8: Preparation of 2-(4-(2-(4-chloro-2-fluorophenyl)-2-methylbenzo[d][1,3]dioxol-4-yl)benzyl)-1-((4-propyl-4H-1,2,4-triazol-3-yl)methyl)-1H-benzo[d]imidazol-6-carboxylic acid]

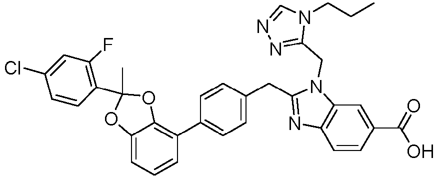

### 2-(4-(2-(4-chloro-2-fluorophenyl)-2-methylbenzo[d][1,3]dioxol-4-yl)benzyl)-1-((4-propyl-4H-1,2,4-triazol-3-yl)methyl)-1H-benzo[d]imidazol-6-carboxylic acid

2-(4-(2-(4-chloro-2-fluorophenyl)-2-methylbenzo[*d*][1,3]dioxol-4-yl)phenyl)acetic acid (100 mg, 0.251 mmol) obtained in Preparation Example 11 was dissolved in DMF (2 mL), and then methyl 4-amino-3-(((4-propyl-4*H*-1,2,4-triazol-3-yl)methyl)amino)benzoate (73 mg, 0.251 mmol) obtained in Preparation Example 17, EDC (96 mg, 0.501 mmol), and HOBt (77 mg, 0.501 mmol) were added. The reactant was stirred at room temperature under nitrogen for 24 h. After completion of the reaction, water was added, extracted with EtOAc, dried over MgSO₄, and concentrated under reduced pressure to obtain the intermediate methyl 4-(2-(4-(2-(4-chloro-2-fluorophenyl)-2-methylbenzo[*d*][1,3]dioxol-4-yl)phenyl)acetamido)-3-(((4-propyl-4*H*-1,2,4-triazol-3-yl)methyl)amino)benzoate (103 mg, 0.154 mmol). Without further purification, it was dissolved in AcOH (0.9 mL, 15.37 mmol) and stirred at 120 °C for 3 h. After completion of the reaction, it was concentrated under reduced pressure. Water was added, extracted with EtOAc, then dried over MgSO₄ and concented under reduced pressure to obtain the intermediate methyl 2-(4-(2-(4-chloro-2-fluorophenyl)-2-methylbenzo[*d*][1,3]dioxol-4-yl)benzyl)-1-((4-propyl-4*H*-1,2,4-triazol-3-yl)methyl)-1*H-*benzo[d]imidazol-6-carboxylate (60 mg, 0.092 mmol). Without further purification, it was dissolved in THF/H₂O (1 mL/1 mL), then NaOH (11 mg, 0.276 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave 2-(4-(2-(4-chloro-2-fluorophenyl)-2-methylbenzo[d][1,3]dioxol-4-yl)benzyl)-1-((4-propyl-4H-1,2,4-triazol-3-yl)methyl)-1H-benzo[d]imidazol-6-carboxylic acid (26 mg, 0.041 mmol, 44%).

¹H NMR (500 MHz, MeOD) δ 8.40 (s, 1H), 8.20 (s, 1H), 8.03 (d, *J* = 8.5 Hz, 1H), 7.76 (d, *J= 7.9* Hz, 1H), 7.67 (d, *J =* 8.2 Hz, 2H), 7.60 (t, *J =* 8.2 Hz, 1H), 7.30-7.33 (m, 3H), 7.24 (d, *J* = 8.2 Hz, 1H), 7.07 (d, *J =* 8.2 Hz, 1H), 6.93 (t, *J =* 7.8 Hz, 1H), 6.85 (d, *J =* 7.6 Hz, 1H), 5.77 (s, 2H), 4.49 (s, 2H), 3.83 (t, *J =* 7.5 Hz, 2H), 2.09 (s, 3H), 1.51 (d, *J =* 7.6 Hz, 2H), 0.75 (t, *J =* 6.6 Hz, 3H).

### [Example 9: Preparation of 2-(4-(2-(4-chloro-2-fluorophenyl)-2-methylbenzo[d][1,3]dioxol-4-yl)-3-fluorobenzyl)-1-((4-propyl-4H-1,2,4-triazol-3-yl)methyl)-1H-benzo[d]imidazol-6-carboxylic acid]

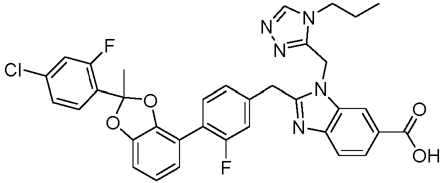

### 2-(4-(2-(4-chloro-2-fluorophenyl)-2-methylbenzo[d][1,3]dioxol-4-yl)-3-fluorobenzyl)-1-((4-propyl-4H-1,2,4-triazol-3-yl)methyl)-1H-benzo[d]imidazol-6-carboxylic acid

2-(4-(2-(4-chloro-2-fluorophenyl)-2-methylbenzo[*d*][1,3]dioxol-4-yl)-3-fluorophenyl)acetic acid (100 mg, 0.240 mmol) obtained in Preparation Example 13 was dissolved in DMF (2 mL), and then methyl 4-amino-3-(((4-propyl-4*H*-1,2,4-triazol-3-yl)methyl)amino)benzoate (69 mg, 0.240 mmol) obtained in Preparation Example 17, EDC (92 mg, 0.480 mmol), and HOBt (74 mg, 0.480 mmol) were added. The reactant was stirred at room temperature under nitrogen for 24 h. After completion of the reaction, water was added, extracted with EtOAc, dried over MgSO₄, and concentrated under reduced pressure to obtain the intermediate methyl 4-(2-(4-(2-(4-chloro-2-fluorophenyl)-2-methylbenzo[*d*][1,3]dioxol-4-yl)-3-fluorophenyl)acetamido)-3-(((4-propyl-4*H*-1,2,4-triazol-3-yl)methyl)amino)benzoate (118 mg, 0.171 mmol). Without further purification, it was dissolved in AcOH (0.98 mL, 17.15 mmol) and stirred at 120 °C for 3 h. After completion of the reaction, it was concentrated under reduced pressure. Water was added, extracted with EtOAc, then dried over MgSO₄ and concented under reduced pressure to obtain the intermediate methyl 2-(4-(2-(4-chloro-2-fluorophenyl)-2-methylbenzo[*d*][1,3]dioxol-4-yl)-3-fluorobenzyl)-1-((4-propyl-4*H*-1,2,4-triazol-3-yl)methyl)-1*H*-benzo[*d*]imidazol-6-carboxylate (70 mg, 0.104 mmol). Without further purification, it was dissolved in THF/H₂O (1 mL/1 mL), then NaOH (13 mg, 0.313 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave 2-(4-(2-(4-chloro-2-fluorophenyl)-2-methylbenzo[*d*][1,3]dioxol-4-yl)-3-fluorobenzyl)-1-((4-propyl-4*H*-1,2,4-triazol-3-yl)methyl)-1*H*-benzo[*d*]imidazol-6-carboxylic acid (27 mg, 0.041 mmol, 39%).

¹H NMR (500 MHz, MeOD) δ 8.43 (s, 1H), 8.24 (s, 1H), 8.04 (d, *J* = 8.2 Hz, 1H), 7.88 (d, *J* = 8.5 Hz, 1H), 7.77 (t, *J =* 9.5 Hz, 2H), 7.64 (d, *J =* 8.2 Hz, 1H), 7.40 (s, 1H), 7.31 (d, *J =* 11.0 Hz, 1H), 7.24 (d, *J =* 8.5 Hz, 1H), 7.11 (d, *J =* 9.5 Hz, 1H), 6.90-6.93 (m, 2H), 5.83 (s, 2H), 4.51 (s, 2H), 3.90 (t, *J =* 7.3 Hz, 2H), 2.05 (s, 3H), 1.55 (d, *J =* 7.9 Hz, 2H), 0.78 (t, *J=* 7.3 Hz, 3H).

### [Example 10: Preparation of 2-(4-(2-(4-chloro-2-fluorophenyl)-2-methylbenzo[d][1,3]dioxol-4-yl)benzyl)-1-((1-ethyl-1H-imidazol-5-yl)methyl)-1H-benzo[d]imidazol-6-carboxylic acid]

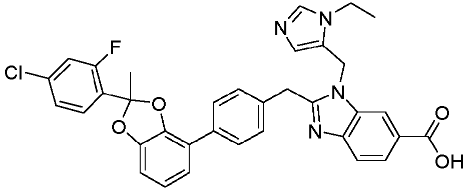

### 2-(4-(2-(4-chloro-2-fluorophenyl)-2-methylbenzo[d][1,3]dioxol-4-yl)benzyl)-1-((1-ethyl-1H-imidazol-5-yl)methyl)-1H-benzo[d]imidazol-6-carboxylic acid

2-(4-(2-(4-chloro-2-fluorophenyl)-2-methylbenzo[*d*][1,3]dioxol-4-yl)phenyl)acetic acid (155 mg, 0.389 mmol) obtained in Preparation Example 11 was dissolved in DMF (2 mL), and then methyl 4-amino-3-(((1-ethyl-1H-imidazol-5-yl)methyl)amino)benzoate (107 mg, 0.389 mmol) obtained in Preparation Example 4, EDC (149 mg, 0.777 mmol), and HOBt (119 mg, 0.777 mmol) were added. The reactant was stirred at room temperature under nitrogen for 24 h. After completion of the reaction, water was added, extracted with EtOAc, dried over MgSO₄, and concentrated under reduced pressure to obtain the intermediate methyl 4-(2-(4-(2-(4-chloro-2-fluorophenyl)-2-methylbenzo[*d*][1,3]dioxol-4-yl)phenyl)acetamido)-3-(((1-ethyl-1H-imidazol-5-yl)methyl)amino)benzoate (129 mg, 0.197 mmol). Without further purification, it was dissolved in AcOH (1.1 mL, 15.37 mmol) and stirred at 120 °C for 3 h. After completion of the reaction, it was concentrated under reduced pressure. Water was added, extracted with EtOAc, then dried over MgSO₄ and concented under reduced pressure to obtain the intermediate methyl 2-(4-(2-(4-chloro-2-fluorophenyl)-2-methylbenzo[*d*][1,3]dioxol-4-yl)benzyl)-1-((1-ethyl-1*H*-imidazol-5-yl)methyl)-1*H-*benzo[*d*]imidazol-6-carboxylate (116 mg, 0.182 mmol). Without further purification, it was dissolved in THF/H₂O (1 mL/1 mL), then NaOH (22 mg, 0.546 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave 2-(4-(2-(4-chloro-2-fluorophenyl)-2-methylbenzo[*d*][1,3]dioxol-4-yl)benzyl)-1-((1-ethyl-1*H*-imidazol-5-yl)methyl)-1*H-*benzo[*d*]imidazol-6-carboxylic acid (13 mg, 0.021 mmol, 11%).

¹H NMR (500 MHz, MeOD) δ 8.11 (d, *J* = 9.8 Hz, 1H), 7.98-8.02 (m, 1H), 7.75 (d, *J* = 8.5 Hz, 1H), 7.68 (t, *J =* 8.2 Hz, 3H), 7.58 (t, *J =* 8.2 Hz, 1H), 7.29-7.34 (m, 3H), 7.21 (dd, *J =* 8.4, 1.7 Hz, 1H), 7.06 (d, *J* = 7.9 Hz, 1H), 6.91 (t, *J =* 7.9 Hz, 1H), 6.84 (d, *J =* 7.9 Hz, 1H), 6.51 (s, 1H), 5.58 (s, 2H), 4.44-4.53 (m, 2H), 3.81-3.89 (m, 2H), 2.06 (d, *J =* 12.8 Hz, 3H), 1.15-1.22 (m, 3H).

### [Example 11: Preparation of 2-(4-(2-(4-chloro-2-fluorophenyl)-2-methylbenzo[d][1,3]dioxol-4-yl)-3-fluorobenzyl)-1-((1-ethyl-1H-imidazol-5-yl)methyl)-1H-benzo[d]imidazol-6-carboxylic acid]

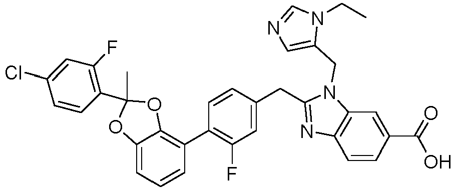

### 2-(4-(2-(4-chloro-2-fluorophenyl)-2-methylbenzo[d][1,3]dioxol-4-yl)-3-fluorobenzyl)-1-((1-ethyl-1H-imidazol-5-yl)methyl)-1H-benzo[d]imidazol-6-carboxylic acid

2-(4-(2-(4-chloro-2-fluorophenyl)-2-methylbenzo[*d*][1,3]dioxol-4-yl)-3-fluorophenyl)acetic acid (103 mg, 0.247 mmol) obtained in Preparation Example 41 was dissolved in DMF (2 mL), and then methyl 4-amino-3-(((1-ethyl-1H-imidazol-5-yl)methyl)amino)benzoate (68 mg, 0.247 mmol) obtained in Preparation Example 4, EDC (95 mg, 0.494 mmol), and HOBt (76 mg, 0.494 mmol) were added. The reactant was stirred at room temperature under nitrogen for 24 h. After completion of the reaction, water was added, extracted with EtOAc, dried over MgSO₄, and concentrated under reduced pressure to obtain the intermediate methyl 4-(2-(4-(2-(4-chloro-2-fluorophenyl)-2-methylbenzo[*d*][1,3]dioxol-4-yl)-3-fluorophenyl)acetamido)-3-(((1-ethyl-1*H*-imidazol-5-yl)methyl)amino)benzoate (109 mg, 0.162 mmol). Without further purification, it was dissolved in AcOH (0.93 mL, 16.19 mmol) and stirred at 120 °C for 3 h. After completion of the reaction, it was concentrated under reduced pressure. Water was added, extracted with EtOAc, then dried over MgSO₄ and concented under reduced pressure to obtain the intermediate methyl 2-(4-(2-(4-chloro-2-fluorophenyl)-2-methylbenzo[*d*][1,3]dioxol-4-yl)-3-fluorobenzyl)-1-((1-ethyl-1*H*-imidazol-5-yl)methyl)-1*H*-benzo[*d*]imidazol-6-carboxylate (90 mg, 0.137 mmol). Without further purification, it was dissolved in THF/H₂O (1 mL/1 mL), then NaOH (16 mg, 0.412 mmol) was added and stirred at room temperature for 24 h. After completion of the reaction, water was added and acidified to pH ~2 with 1N HCl. It was extracted with EtOAc, dried over MgSO₄ and concentrated under reduced pressure. MPLC gave 2-(4-(2-(4-chloro-2-fluorophenyl)-2-methylbenzo[*d*][1,3]dioxol-4-yl)-3-fluorobenzyl)-1-((1-ethyl-1*H*-imidazol-5-yl)methyl)-1*H-*benzo[d]imidazol-6-carboxylic acid (19 mg, 0.030 mmol, 22%).

¹H NMR (500 MHz, MeOD) δ 8.15 (s, 1H), 8.03 (d, *J =* 8.2 Hz, 1H), 7.76 (d, *J =* 8.5 Hz, 1H), 7.60-7.64 (m, 2H), 7.41 (t, *J =* 7.8 Hz, 1H), 7.30 (dd, *J =* 11.0, 2.1 Hz, 1H), 7.23 (d, *J =* 8.2 Hz, 1H), 7.06-7.10 (m, 2H), 6.87-6.93 (m, 3H), 6.51 (s, 1H), 5.63 (s, 2H), 4.49 (s, 2H), 3.90 (q, *J =* 7.2 Hz, 2H), 2.04 (d, *J =* 7.0 Hz, 3H), 1.21 (t, *J =* 7.3 Hz, 3H).

### [Example 12: Preparation of 2-(4-(2-(4-chloro-2-fluorophenyl)-2-methylbenzo[d][1,3]dioxol-4-yl)benzyl)-1-(((S)-tetrahydrofuran-2-yl)methyl)-1H-benzo[d]imidazol-6-carboxylic acid]

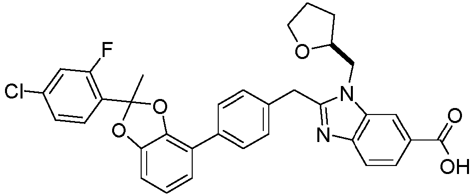

### 2-(4-(2-(4-chloro-2-fluorophenyl)-2-methylbenzo[d][1,3]dioxol-4-yl)benzyl)-1-(((S)-tetrahydrofuran-2-yl)methyl)-1H-benzo[d]imidazol-6-carboxylic acid

Methyl (*S*)-4-amino-3-(((tetrahydrofuran-2-yl)methyl)amino)benzoate (50.5 mg, 0.202 mmol) obtained in Preparation Example 8 and 2-(4-(2-(4-chloro-2-fluorophenyl)-2-methylbenzo[*d*][1,3]dioxol-4-yl)phenyl)acetic acid (89 mg, 0.222 mmol) obtained in Preparation Example 39 were dissolved in 5 mL of DMF, and then EDC (77 mg, 0.404 mmol), and HOBt (61.8 mg, 0.404 mmol) were added and stirred at room temperature for 15 h. After completion of the reaction, water was added, and extracted with EtOAc. The organic layer was dried over Na₂SO₄, concentrated under reduced pressure, and purified by MPLC to obtain the intermediate methyl 4-(2-(4-(2-(4-chloro-2-fluorophenyl)-2-methylbenzo[*d*][1,3]dioxol-4-yl)phenyl)acetamido)-3-((((*S*)-tetrahydrofuran-2-yl)methyl)amino)benzoate. Without further purification, it was dissolved in 10 mL of acetic acid and stirred at 120 °C for 2 h. Acetic acid was concentrated under reduced pressure and purified by MPLC to obtain the intermediate methyl 2-(4-(2-(4-chloro-2-fluorophenyl)-2-methylbenzo[*d*][1,3]dioxol-4-yl)benzyl)-1-(((*S*)-tetrahydrofuran-2-yl)methyl)-1*H*-benzo[*d*]imidazol-6-carboxylate. The intermediate was dissolved in 5 mL of THF, 0.5 mL of 1N NaOH was added to the reactant and stirred at 50 °C for 15 h. 1N HCl was added, pH was adjusted to 4, and extracted with EtOAc. The organic layer was dried over Na₂SO₄, concentrated under reduced pressure, and purified by MPLC to obtain 2-(4-(2-(4-chloro-2-prolophenyl)-2-methylbenzo[*d*][1,3]dioxol-4-yl)benzyl)-1-(((*S*)-tetrahydrofuran-2-yl)methyl)-1*H*-benzo[*d*]imidazol-6-carboxylic acid (35 mg, 0.058 mmol, 28.7%).
¹H NMR (400 MHz, CDCl₃) δ 8.18 (s, 1H), 8.08 (dd, *J* = 8.5, 1.1 Hz, 1H), 7.87 (d, *J*= 8.2 Hz, 1H), 7.70 (d, *J =* 8.2 Hz, 2H), 7.50 (t, *J* = 8.2 Hz, 1H), 7.36 (d, *J =* 8.2 Hz, 2H), 7.12 (dd, *J =* 10.5, 1.8 Hz, 1H), 7.07 (d, *J* = 8.2 Hz, 1H), 7.01 (d, *J =* 7.8 Hz, 1H), 6.86 (t, *J =* 7.8 Hz, 1H), 6.79 (d, *J =* 7.8 Hz, 1H), 4.56 (dd, *J =* 42.8, 15.8 Hz, 2H), 4.19-4.32 (m, 1H), 4.10 - 4.17 (m, 2H), 3.87 (q, *J* = 7.3 Hz, 1H), 3.70 - 3.76 (m, 1H), 1.98 - 2.06 (m, 4H), 1.82 - 1.89 (m, 2H), 1.52 - 1.59 (m, 1H); LC-MS(ESI): 599.33 [M+H]⁺

### [Example 13: Preparation of (S)-2-(4-(6-(bicyclo[2.2.2]octan-1-ylmethoxy)pyridin-2-yl)benzyl)-1-(oxetan-2-ylmethyl)-1H-benzo[d]imidazol-6-carboxylic acid]

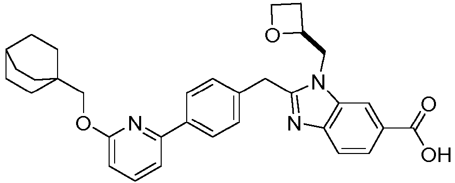

### (S)-2-(4-(6-(bicycli[2.2.2]octan-1-ylmethoxy)pyridin-2-yl)benzyl)-1-(oxetan-2-ylmethyl)-1H-benzo[d]imidazol-6-carboxylic acid

Methyl (*S*)-2-(4-(6-(bicyclo[2.2.2]octan-1-ylmethoxy)pyridin-2-yl)benzyl)-1-(oxytan-2-ylmethyl)-1*H*-benzo[*d*]imidazol-6-carboxylate (110 mg, 0.199 mmol) obtained in Preparation Example 22 was dissolved in 5 mL of THF, 0.5 mL of 1N NaOH was added to the reaction and stirred at room temperature for 15 h. After completion of the reaction, water was added, the pH was adjusted to 4 with 1N-HCl, extracted with EtOAc, dried over Na₂SO₄, and concentrated under reduced pressure. MPLC gave (*S*)-2-(4-(6-(bicyclo[2.2.2]octan-1-ylmethoxy)pyridin-2-yl)benzyl)-1-(oxytan-2-ylmethyl)-1*H* benzo[*d*]imidazol-6-carboxylic acid (45 mg, 0.084 mmol, 42%).
¹H NMR (500 MHz, CDCl₃) δ 8.14 (s, 1H), 8.07 (d, *J =* 8.5 Hz, 1H), 7.96 (d, *J =* 8.2 Hz, 2H), 7.86 (d, *J =* 8.5 Hz, 1H), 7.56 (t, *J =* 7.8 Hz, 1H), 7.34 (d, *J =* 8.2 Hz, 2H), 7.23 (d, *J* = 7.6 Hz, 1H), 6.63 (d, *J =* 8.2 Hz, 1H), 5.05 - 5.08 (m, 1H), 4.53 - 4.63 (m, 3H), 4.32 - 4.40 (m, 2H), 4.25 (dd, *J =* 15.6, 2.7 Hz, 1H), 3.99 (s, 2H), 2.61 - 2.68 (m, 1H), 2.29 - 2.36 (m, 1H), 1.49 - 1.60 (m, 13H); LC-MS(ESI): 538.38 [M+H]⁺

### [Experimental Example 1: GLP-1R agonist activity measurement (Cell-based Luciferase Assay)]

The CHO-K1/hGLP-1R/CRE-luciferase cell line was seeded at 15000 cells in a 96-well plate. As a medium, Ham's F-12 Nutrient medium was used. After culturing for 18 hours in a 5% CO₂ incubator maintained at 37 °C, the drug was treated. Each drug plated at 9 concentrations was dissolved in DMEM/F-12 + 1% FBS medium and treated with 100 ul of each cell line. After culturing for 4 h, 30 ul of assay reagent was added to each well using the Bright-Glo^{™} luficerase assay system kit. After the plate was stored at room temperature for 15 min, luminoscence was measured with a SpectraMax M5 instrument. The GLP-1R activity of the Example compound obtained through the above experiment in EC₅₀ (nM) unit is shown in Table 1 below.

**[Table 1]**

| **Examples** | **EC₅₀** | **Examples** | **EC₅₀** |
|---|---|---|---|
| 1 | 0.316 | 2 | 0.108 |
| 3 | 0.041 | 4 | 0.278 |
| 5 | 0.16 | 6 | 3.5 |
| 7 | >80 | 8 | 0.914 |
| 9 | 0.772 | 10 | 0.0216 |
| 11 | 0.0256 | 12 | 0.223 |
| 13 | >80 | | |

As may be seen in Table 1, the compounds corresponding to Chemical Formula 1, Chemical Formula 1' or Chemical Formula 1" according to the present disclosure were confirmed to have excellent effects as excellent GLP-1 receptor agonists. In addition, a compounds corresponding to Chemical Formula 1 were confirmed to have an excellent DMPK profile through drug metabolism-related experiments and pharmacokinetics-related experiments such as Cytochrome P (CYP) inhibition/induction experiments, metabolic stability (MS) experiments, and the like.

Further disclosed are the following embodiments:
1. A compound of the following Chemical Formula I, an isomer thereof, or a pharmaceutically acceptable salt thereof: 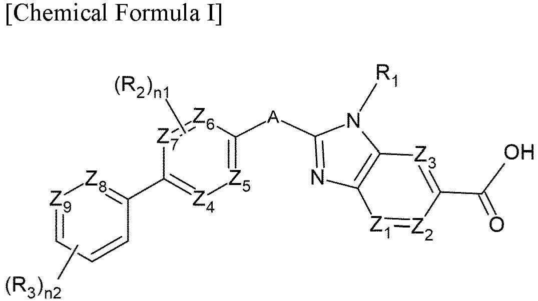
   A is -(CH₂)ₘ-, -O- or -N(Rₐ)-, wherein m is an integer from 1 to 3 and Rₐ is hydrogen or alkyl;
   Z₁, Z₂, Z₃, Z₄, Z₅, Z₆ or Z₇ each independently represents CH, CF, CCl, CBr, CI or N;
   Z₈ or Z₉ is each independently C or N substituted with an -O-CH₂-R group, or when Z₈ and Z₉ are both C, each substituent may be condensed with each other to form a dioxole structure;
   R is cycloalkyl or 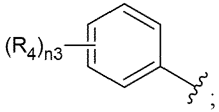
   R₁ is (cycloalkyl)alkyl, (heterocycloalkyl)alkyl, (aryl)alkyl or (heteroaryl)alkyl;
   R₂, R₃ or R₄ is each independently a hydrogen, deuterium, halo, alkyl, alkoxy, alkylamine or nitrile group;
   n₁ to n₃ are each independently an integer of 1 to 4, wherein, when n₁ to n₃ are an integer of 2 or more, each of R₂, R₃ or R₄ may be the same as or different from each other; and
   at this time, the alkyl, alkoxy, alkylamine, cycloalkyl, heterocycloalkyl, aryl, or heteroaryl is unsubstituted or substituted.
2. The compound, the isomer thereof, or the pharmaceutically acceptable salt thereof of embodiment 1, wherein the compound of the following Chemical Formula I is a compound represented by the following Chemical Formula 1, Chemical Formula 1' or Chemical Formula 1": 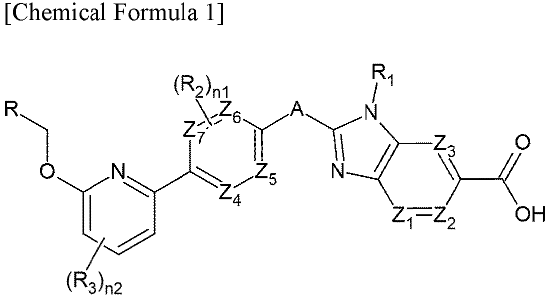 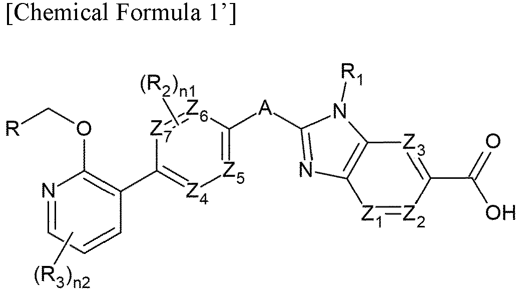 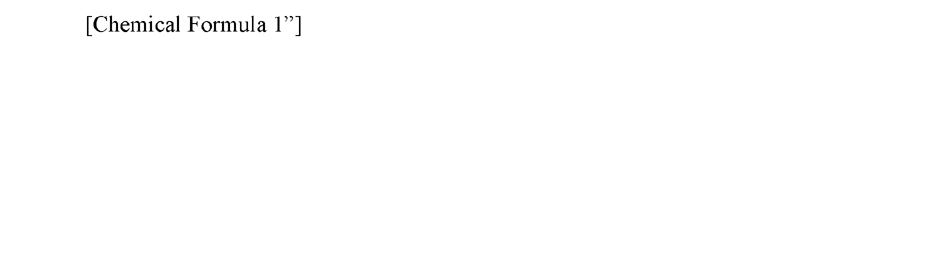 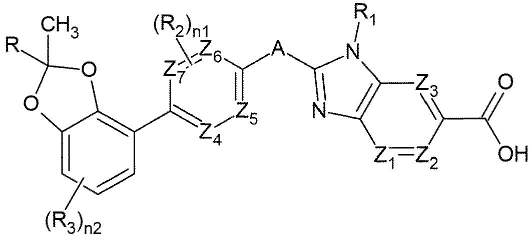 A, Z₁, Z₂, Z₃, Z₄, Z₅, Z₆, Z₇, R, R₁, R₂, R₃, R₄ and n₁ to n₃ are as defined in embodiment 1.
3. The compound, the isomer thereof, or the pharmaceutically acceptable salt thereof of embodiment 1, wherein A is -CH₂-, -O- or -N(Rₐ)-, wherein Rₐ is hydrogen or C₁₋₃ alkyl.
4. The compound, the isomer thereof, or the pharmaceutically acceptable salt thereof of embodiment 1,
   wherein, R is C₃₋₈ cycloalkyl or 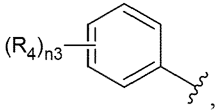
   R₁ may be (C₃₋₈ cycloalkyl)C₁₋₃ alkyl, (4- to 10-membered heterocycloalkyl)C₁₋₃ alkyl, (C₆₋₁₀ aryl)alkyl or (4- to 10-membered heteroaryl)C₁₋₃ alkyl, wherein the heterocycloalkyl or heteroaryl contains 1 to 3 heteroatoms selected from the group consisting of N, O and S; and
   R₂, R₃ or R₄ is each independently hydrogen, deuterium, F, Cl, Br, I, C₁₋₃ alkyl, C₁₋₃ alkoxy, C₁₋₃ alkylamine or nitrile group, n₁ to n₃ are each independently an integer of 1 to 3, and when n₁ to n₃ is an integer of 2 or more, each of R₂, R₃ or R₄ may be the same as or different from each other.
5. The compound, the isomer thereof, or the pharmaceutically acceptable salt thereof of embodiment 1, wherein Z₁, Z₂, Z₃, Z₄, Z₅, Z₆ or Z₇ are each independently CH, CF or CCl.
6. The compound, the isomer thereof, or the pharmaceutically acceptable salt thereof of embodiment 1, wherein R is bicyclo[1,1,0]butane, bicyclo[1,1,1]pentane, bicyclo[2,1,1]hexane, bicyclo[2,2,1]heptane, bicyclo[2,2,2]octane or 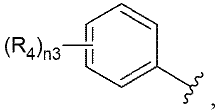 and
   R₁ is cyclopropylmethyl, cyclobutylmethyl, cyclopentylmethyl, cyclohexylmethyl, oxytanylmethyl, tetrahydrofuranylmethyl, tetrahydropyranylmethyl, oxazolylmethyl, benzyl, unsubstituted or triazolylmethyl substituted with propyl, or unsubstituted or imidazolylmethyl substituted with ethyl.
7. The compound, the isomer thereof, or the pharmaceutically acceptable salt thereof of embodiment 1, wherein the compound represented by Chemical Formula I is selected from the group consisting of the following compounds:
   1] (*S*)-2-(4-(2-((4-chloro-2-fluorobenzyl)oxy)pyridin-3-yl)benzyl)-1-(oxytan-2-ylmethyl)-1*H*-benzo[d]imidazol-6-carboxylic acid;
   2] 2-(4-(2-(4-chloro-2-fluorophenyl)-2-methylbenzo[*d*][1,3]dioxol-4-yl)benzyl)-1-(((*S*)-oxytan-2-yl)methyl)-1*H*-benzo[*d*]imidazol-6-carboxylic acid;
   3] 2-(4-(2-(4-chloro-2-fluorophenyl)-2-methylbenzo[*d*][1,3]dioxol-4-yl)-3-fluorobenzyl)-1-(((*S*)-oxytan-2-yl)methyl)-1*H*-benzo[*d*]imidazol-6-carboxylic acid;
   4] 2-(4-(2-(4-chloro-2-fluorophenyl)-2-methylbenzo[*d*][1,3]dioxol-4-yl)benzyl)-1-(oxazol-5-ylmethyl)-1*H*-benzo[*d*]imidazol-6-carboxylic acid;
   5] 2-(4-(2-(4-chloro-2-fluorophenyl)-2-methylbenzo[*d*][1,3]dioxol-4-yl)-3-fluorobenzyl)-1-(oxazol-5-ylmethyl)-1*H*-benzo[d]imidazol-6-carboxylic acid;
   6] 2-(4-(2-((4-chloro-2-fluorobenzyl)oxy)pyridin-3-yl)benzyl)-1-(oxazol-5-ylmethyl)-1*H*-benzo[d]imidazol-6-carboxylic acid;
   7] 2-(4-(2-((4-chloro-2-fluorobenzyl)oxy)pyridin-3-yl)benzyl)-1-((4-propyl-4*H*-1,2,4-triazol-3-yl)methyl)-1*H*-benzo[d]imidazol-6-carboxylic acid;
   8] 2-(4-(2-(4-chloro-2-fluorophenyl)-2-methylbenzo[*d*][1,3]dioxol-4-yl)benzyl)-1-((4-propyl-4*H*-1,2,4-triazol-3-yl)methyl)-1*H*-benzo[d]imidazol-6-carboxylic acid;
   9] 2-(4-(2-(4-chloro-2-fluorophenyl)-2-methylbenzo[*d* [1,3]dioxol-4-yl)-3-fluorobenzyl)-1-((4-propyl-4*H*-1,2,4-triazol-3-yl)methyl)-1*H*-benzo[*d*]imidazol-6-carboxylic acid;
   10] 2-(4-(2-(4-chloro-2-fluorophenyl)-2-methylbenzo[*d*][1,3]dioxol-4-yl)benzyl)-1-((1-ethyl-1*H*-imidazol-5-yl)methyl)-1*H*-benzo[d]imidazol-6-carboxylic acid;
   11] 2-(4-(2-(4-chloro-2-fluorophenyl)-2-methylbenzo[*d*][1,3]dioxol-4-yl)-3-fluorobenzyl)-1-((1-ethyl-1*H*-imidazol-5-yl)methyl)-1*H*-benzo[d]imidazol-6-carboxylic acid;
   12] 2-(4-(2-(4-chloro-2-fluorophenyl)-2-methylbenzo[*d*][1,3]dioxol-4-yl)benzyl)-1-(((*S*)-tetrahydrofuran-2-yl)methyl)-1*H*-benzo[*d*]imidazol-6-carboxylic acid; and
   13] (*S*)-2-(4-(6-(bicyclo[2.2.2]octan-1-ylmethoxy)pyridin-2-yl)benzyl)-1-(oxytan-2-ylmethyl)-1*H*-benzo[*d*]imidazol-6-carboxylic acid.
8. A pharmaceutical composition for the prevention or treatment of metabolic or neurodegenerative diseases, the pharmaceutical composition comprising a compound of any one of embodiments 1 to 7, an isomer thereof or a pharmaceutically acceptable salt thereof, and a pharmaceutically acceptable excipient, diluent or carrier.
9. The pharmaceutical composition of embodiment 8, wherein the metabolic disease is selected from the group consisting of diabetes, hypertension, hypoglycemia, hyperlipidemia (dyslipidemia), atherosclerosis, coronary artery disease, cardiovascular disorder, blood coagulation abnormality, obesity, diabetic complication, diabetic retinopathy, liver disease, hepatobiliary disease, fatty liver, alcoholic steatohepatitis, chronic kidney disease, insulin resistance, and impaired glucose tolerance.
10. The pharmaceutical composition of embodiment 8, wherein the neurodegenerative disease is selected from the group consisting of Parkinson's disease and Alzheimer's disease.
11. A method for preparing a compound of the following Chemical Formula 1, comprising:
   1) reacting a compound of the following Chemical Formula 2 with a compound of the following Chemical Formula 3 under a palladium catalyst to obtain a compound of the following Chemical Formula 4;
   2) reacting the compound of the following Chemical Formula 4 obtained in step 1) with a compound of the following Chemical Formula 5 under a palladium catalyst and then hydrolyzing to obtain a compound of the following Chemical Formula 6; and
   3) a coupling reaction of the compound of the following Chemical Formula 6 obtained in step 2) with a compound of the following Chemical Formula 7, followed by condensation and hydrolysis to obtain a compound of the following Chemical Formula 1; 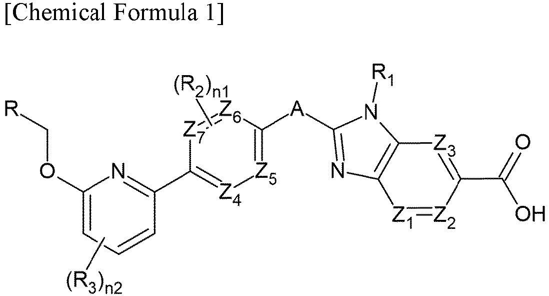 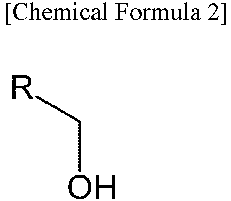 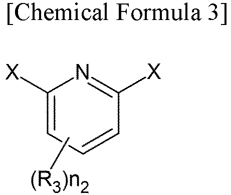 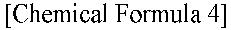 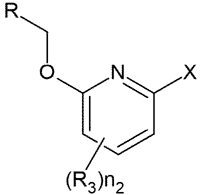 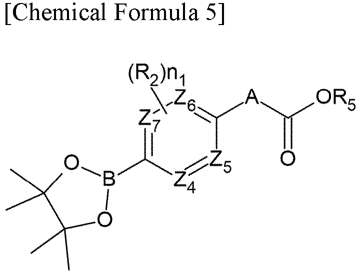 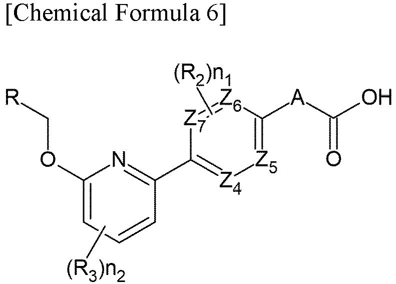 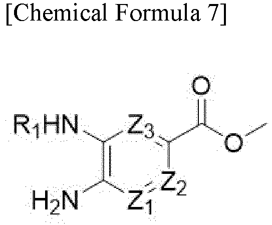 wherein,
      A is carbon;
      n₁, n₂, n₃, R, R₁, R₂, R₃, R₄, Z₁, Z₂, Z₃, Z₄, Z₅, Z₆ and Z₇ are as defined in embodiment 1;
      R₅ is alkyl; and
      X is halo.
12. A method for preparing a compound of the following Chemical Formula 1', comprising:
   1') reacting a compound of the following Chemical Formula 3' with a compound of the following Chemical Formula 5 under a palladium catalyst to obtain a compound of the following Chemical Formula 8;
   2') reacting the compound of the following Chemical Formula 8 obtained in step 1') with a compound of the following Chemical Formula 2 to obtain a compound of the following Chemical Formula 6'; and
   3') a coupling reaction of the compound of the following Chemical Formula 6' obtained in step 2') with a compound of the following Chemical Formula 7, followed by condensation and hydrolysis to obtain a compound of the following Chemical Formula 1'; 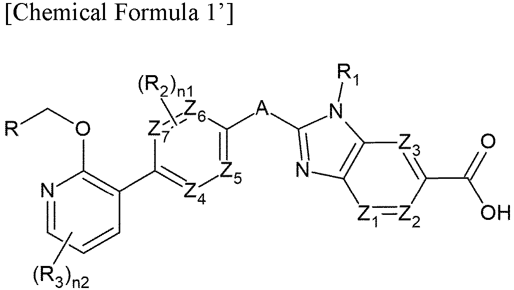 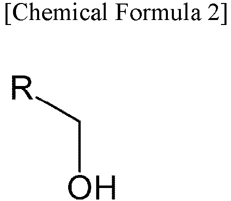 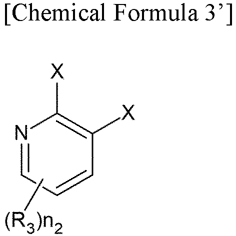 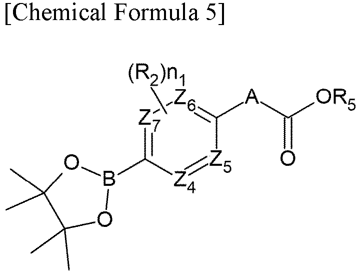 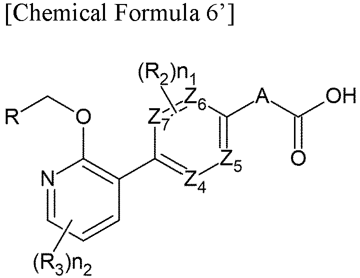 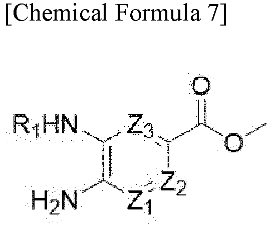 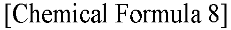 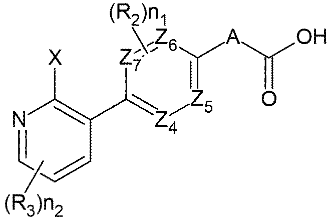 wherein,
      A is carbon;
      n₁, n₂, n₃, R, R₁, R₂, R₃, R₄, Z₁, Z₂, Z₃, Z₄, Z₅, Z₆ and Z₇ are as defined in embodiment 1;
      R₅ is alkyl; and
      X is halo.
13. A method for preparing a compound of the following Chemical Formula 1", comprising:
   1") reacting a compound of the following Chemical Formula 2' with a compound of the following Chemical Formula 3' to obtain a compound of the following Chemical Formula 4';
   2") reacting the compound of the following Chemical Formula 4' obtained in step 1") with a compound of the following Chemical Formula 5 under a palladium catalyst and then hydrolyzing to obtain a compound of the following Chemical Formula 6"; and
   3") a coupling reaction of the compound of the following Chemical Formula 6" obtained in step 2") with a compound of the following Chemical Formula 7, followed by condensation and hydrolysis to obtain a compound of the following Chemical Formula 1"; 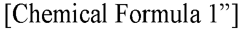 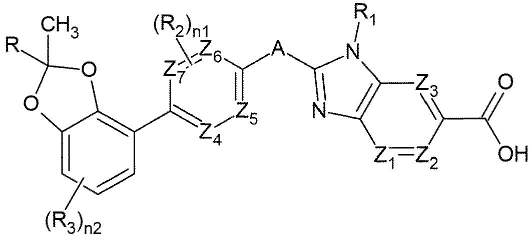 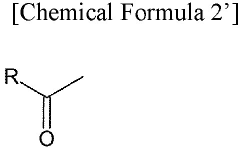 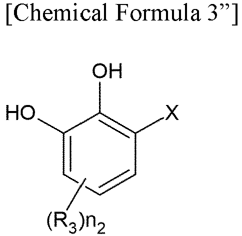 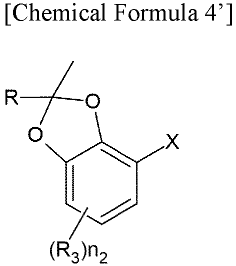 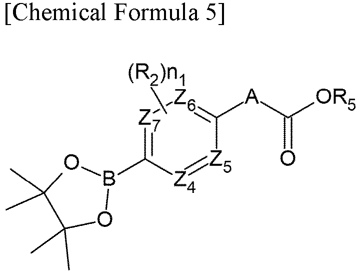 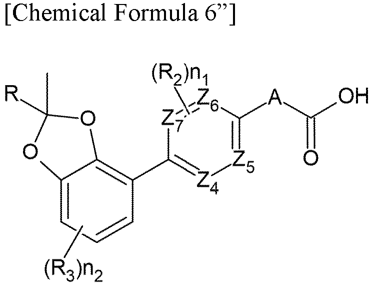 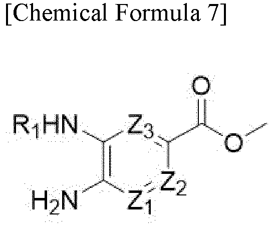 wherein,
      A is carbon;
      n₁, n₂, n₃, R, R₁, R₂, R₃, R₄, Z₁, Z₂, Z₃, Z₄, Z₅, Z₆ and Z₇ are as defined in embodiment 1;
      R₅ is alkyl; and
      X is halo.

## Claims

1. A compound of the following Chemical Formula 1', an isomer thereof, or a pharmaceutically acceptable salt thereof: 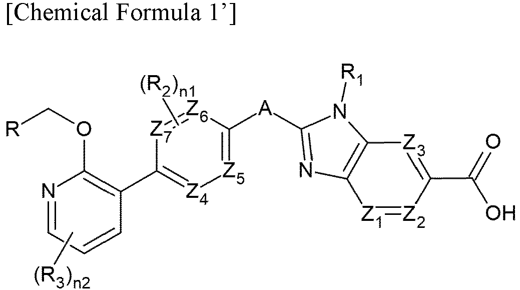 wherein
A is -(CH₂)ₘ-, -O- or -N(Rₐ)-, wherein m is an integer from 1 to 3 and Rₐ is hydrogen or alkyl;
Z₁, Z₂, Z₃, Z₄, Z₅, Z₆ or Z₇ each independently represents CH, CF, CCl, CBr, Cl or N;
R is cycloalkyl or 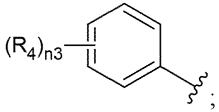
R₁ is (cycloalkyl)alkyl, (heterocycloalkyl)alkyl, (aryl)alkyl or (heteroaryl)alkyl;
R₂, R₃ and R₄ are each independently hydrogen, deuterium, halo, alkyl, alkoxy, alkylamine or nitrile;
n₁ and n₃ are each independently an integer of 1 to 4 and n₂ is an integer of 1 to 3, wherein, when n₁ to n₃ are an integer of 2 or more, each of R₂, R₃ or R₄ may be the same as or different from each other; and
at this time, the alkyl, alkoxy, alkylamine, cycloalkyl, heterocycloalkyl, aryl, or heteroaryl is unsubstituted or substituted.

2. The compound, the isomer thereof, or the pharmaceutically acceptable salt thereof of claim 1, wherein A is -CH₂-, -O- or -N(Rₐ)-, wherein Rₐ is hydrogen or C₁₋₃ alkyl.

3. The compound, the isomer thereof, or the pharmaceutically acceptable salt thereof of claim 1,
wherein, R is C₃₋₈ cycloalkyl or 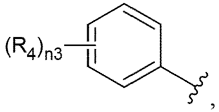
R₁ is (C₃₋₈ cycloalkyl)C₁₋₃ alkyl, (4- to 10-membered heterocycloalkyl)C₁₋₃ alkyl, (C₆₋₁₀ aryl)alkyl or (4- to 10-membered heteroaryl)C₁₋₃ alkyl, wherein the heterocycloalkyl or heteroaryl contains 1 to 3 heteroatoms selected from the group consisting of N, O and S; and
R₂, R₃ oand R₄ are each independently hydrogen, deuterium, F, Cl, Br, I, C₁₋₃ alkyl, C₁₋₃ alkoxy, C₁₋₃ alkylamine or nitrile, n₁ to n₃ are each independently an integer of 1 to 3, and when n₁ to n₃ is an integer of 2 or more, each of R₂, R₃ or R₄ may be the same as or different from each other.

4. The compound, the isomer thereof, or the pharmaceutically acceptable salt thereof of claim 1, wherein Z₁, Z₂, Z₃, Z₄, Z₅, Z₆ and Z₇ are each independently CH, CF or CCl.

5. The compound, the isomer thereof, or the pharmaceutically acceptable salt thereof of claim 1, wherein R is bicyclo[1,1,0]butane, bicyclo[1,1,1]pentane, bicyclo[2,1,1]hexane, bicyclo[2,2,1]heptane, bicyclo[2,2,2]octane or 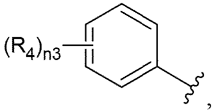 and
R₁ is cyclopropylmethyl, cyclobutylmethyl, cyclopentylmethyl, cyclohexylmethyl, oxytanylmethyl, tetrahydrofuranylmethyl, tetrahydropyranylmethyl, oxazolylmethyl, benzyl, unsubstituted or triazolylmethyl substituted with propyl, or unsubstituted or imidazolylmethyl substituted with ethyl.

6. The compound, the isomer thereof, or the pharmaceutically acceptable salt thereof of claim 1, wherein the compound represented by Chemical Formula 1' is selected from the group consisting of the following compounds:
1] (*S*)-2-(4-(2-((4-chloro-2-fluorobenzyl)oxy)pyridin-3-yl)benzyl)-1-(oxetan-2-ylmethyl)-1*H*-benzo[*d*]imidazol-6-carboxylic acid;
6] 2-(4-(2-((4-chloro-2-fluorobenzyl)oxy)pyridin-3-yl)benzyl)-1-(oxazol-5-ylmethyl)-1*H*-benzo[*d*]imidazol-6-carboxylic acid;
7] 2-(4-(2-((4-chloro-2-fluorobenzyl)oxy)pyridin-3-yl)benzyl)-1-((4-propyl-4*H*-1,2,4-triazol-3-yl)methyl)-1*H*-benzo[*d*]imidazol-6-carboxylic acid.

7. A pharmaceutical composition for use in a method for the prevention or treatment of metabolic or neurodegenerative diseases, the pharmaceutical composition comprising a compound of any one of claims 1 to 6, an isomer thereof or a pharmaceutically acceptable salt thereof, and a pharmaceutically acceptable excipient, diluent or carrier.

8. The pharmaceutical composition for use according to claim 7, wherein the metabolic disease is selected from the group consisting of diabetes, hypertension, hypoglycemia, hyperlipidemia (dyslipidemia), atherosclerosis, coronary artery disease, cardiovascular disorder, blood coagulation abnormality, obesity, diabetic complication, diabetic retinopathy, liver disease, hepatobiliary disease, fatty liver, alcoholic steatohepatitis, chronic kidney disease, insulin resistance, and impaired glucose tolerance.

9. The pharmaceutical composition for use according to claim 7, wherein the neurodegenerative disease is selected from the group consisting of Parkinson's disease and Alzheimer's disease.

10. A method for preparing a compound of the following Chemical Formula 1', comprising:
1') reacting a compound of the following Chemical Formula 3' with a compound of the following Chemical Formula 5 under a palladium catalyst to obtain a compound of the following Chemical Formula 8;
2') reacting the compound of the following Chemical Formula 8 obtained in step 1') with a compound of the following Chemical Formula 2 to obtain a compound of the following Chemical Formula 6'; and
3') a coupling reaction of the compound of the following Chemical Formula 6' obtained in step 2') with a compound of the following Chemical Formula 7, followed by condensation and hydrolysis to obtain a compound of the following Chemical Formula 1'; 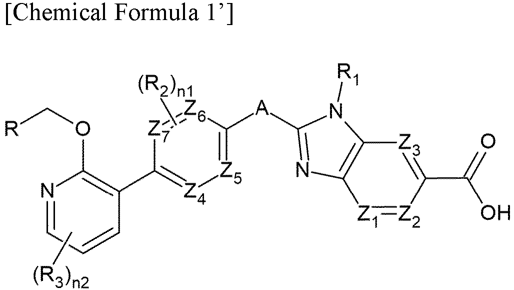 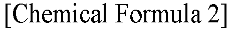 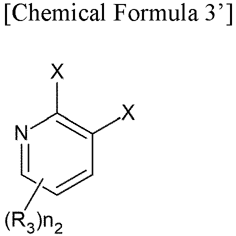 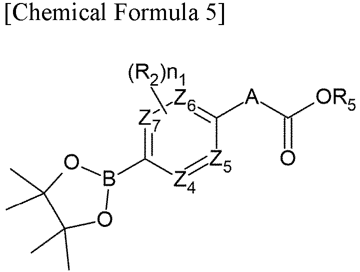 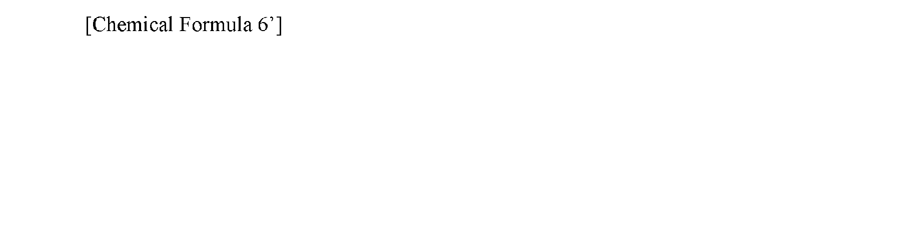 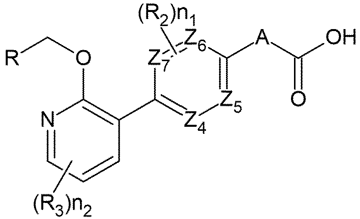 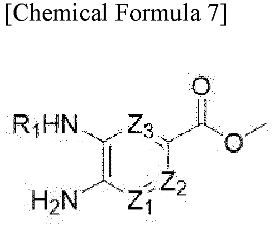 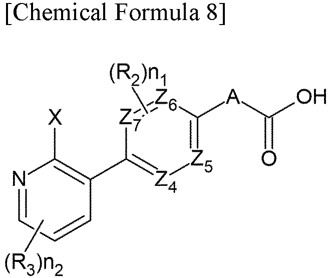 wherein,
A is carbon;
n₁, n₂, n₃, R, R₁, R₂, R₃, R₄, Z₁, Z₂, Z₃, Z₄, Z₅, Z₆ and Z₇ are as defined in claim 1;
R₅ is alkyl; and
X is halo.
